# EUROPEAN PATENT APPLICATION

(11) **EP 4 613 274 A1**
(43) Date of publication of application: **10.09.2025**
(21) Application number: 24161725.7
(22) Date of filing: 06.03.2024
(51) Int. Cl.: A61K 31/53, A61P 35/00

(54) **SENOLYTIC COMPOUNDS**

(71) Applicant: Freie Universität Berlin, 14195 Berlin (DE); Charité - Universitätsmedizin Berlin, 10117 Berlin (DE)
(72) Inventor: RADEMANN, Jörg., 13465 Berlin (DE); KLEMCZAK, Damian., 10551 Berlin, (DE); TIEMANN, Markus., 14199 Berlin (DE); SCHMITT, Clemens., 4048 Puchenau (AT); REIMANN, Maurice., 10711 Berlin (DE)
(74) Representative: Maikowski & Ninnemann Patentanwälte Partnerschaft mbB

(57) **Abstract**

The present invention relates, amongst others, to the use of a compound according to general formula (I), a derivative of such compound according to any of general formulae (II), (III), or (IV), or a pharmaceutically acceptable salt or hydrate of such compound or derivative as senolytic. The invention further relates to selected compounds according to general formula (I), (II), (III), or (IV).

## Description

The present invention relates to the novel use of compounds as senolytic according to the preambles of claims 1 and 10, to novel compounds according to the preamble of claim 11, as well as to a medicament according to claim 15.

Compounds containing the 3-substituted pyrimido-[5,6-e]-1,2,4-triazine-5,7-dione as core structure (general formula (I) have been reported biologically active in multiple bioassays thus complying with the definition of frequent hitters or pan-assay interference compounds (PAINS) (Baell et al., 2010; Baell et al., 2014; Dahlin et al., 2016; Vidler et al., 2018; Baell et al., 2018) The reported bioactivities include antibacterial (Levenberg and Linton, 1966; Latuasan and Berends, 1961; Nagamatsu et al., 1993), antifungal (Li et al., 2019), and anticancer assays (Wei et al., 2010; Raoof et al., 2013; Franci et al., 2017). Further bioactivities as protein target kinases (Hayward et al., 2010), chaperones (Zhou et al., 2009), transcription factor TCF4/β-catenin (Wei et al., 2010; Mao et al., 2014), and tyrosyl-DNA phosphodiesterase 2 (Raoof et al., 2013) have been identified. The core structure was first discovered in the natural product toxoflavin, a toxic metabolite of *Pseudomonas cocovenenans* (Latuasan and Berends, 1961). Therefore, the substances are also referred to as toxoflavins. There have been several accounts on the redox activity of 3-substituted pyrimido-triazines (Light and Walsh, 1980; Stewart et al., 1981; Walsh et al., 1978; Yoneda et al., 1975; Todorovic et al., 2010), however, a detailed and quantitative characterization of the mechanism responsible for activity and a general technical functionality conferred by this mechanism is missing so far.

Yoneda et al. (1971), Yoneda et al. (1973), and Yoneda et al. (1975) describe some of the toxoflavins, their synthesis method, and their reactivity.

JP H09255681 A describes an antitumor agent comprising a 7-azapteridine derivative of a pyrimido-[5,6-e]-1,2,4-triazine-5,7-dione.

Likewise, WO 2010/014798 A2 describes pyrimido-[5,6-e]-1,2,4-triazine-5,7-diones and their use as anti-cancer drug.

DE 103 01 788 A1 describes various pyrimido-[5,6-e]-1,2,4-triazine-5,7-diones, their manufacturing method and their use as drug for treating diabetes.

WO 2012/006104 A2 describes pyrimido-[5,6-e]-1,2,4-triazine-5,7-diones and their use as drug for treating tuberculosis.

WO 2018/140762 A1 describes pyrimido-[5,6-e]-1,2,4-triazine-5,7-diones and their use as drug for treating human immunodeficiency virus (HIV) infections, in particular for inhibiting HIV-1 integrase multimerization.

WO 2010/072807 A2 describes pyrimido-[5,6-e]-1,2,4-triazine-5,7-diones and their use as inhibitors of cystathionine beta synthase which, among other biochemical effects, allow reduction of the neurotoxic overproduction of endogenous hydrogen sulfide.

Senescent cells are cells no longer performing their native biological functions. Similar to apoptosis as another cell-cycle exit program, senescence is a natural cellular stress-responsive mechanism executed when DNA has been damaged or other severe cellular insults were encountered. Senescent cells remain viable and metabolically active, but are unable to proliferate. This condition is entered upon high energy radiation, oxidative stress, chemotherapeutic agents, mutagenesis, and viral infection, among others. Natural aging is also linked to senescence, since after a certain number of divisions, cells stop replicating and enter senescence (replicative senescence). Senescent cells may promote inflammation and chronic disease due to their senescence-associated secretory phenotype (SASP). In tumor therapy, tumor cell (and bystander cell) senescence may occur as an alternative to apoptotic cell death. On the one hand, this is a desired therapeutic effect to stop tumor cell proliferation; on the other hand, senescent cells undergo profound epigenomic reprogramming, which might equip them with latent stemness capacity. Upon occasional cell-cycle re-entry out of senescence, these changes render tumor cells cancer stem cells, accounting for particularly aggressive relapses. The *tumor stem cell program* of those post-senescent cells is a pivotal and detrimental capability to re-initiate tumor growth, translating into poor patient outcome. Accordingly, these previously senescent cells behave more aggressively regarding tumor growth than pre-senescent tumor cells, namely tumor cells that have never been in senescence.

For these reasons, chemical compounds that selectively eliminate senescent cells are urgently needed. Prototypic compounds, called senolytics, are already known, but only a few are currently approved (or about to be approved) on the market. Most known senolytics (i.e., compounds inducing cell death preferentially in senescent cells) exert their senolytic efficacy by inhibiting anti-apoptotic proteins of the Bcl-2 family, which are often found upregulated in stress-surviving senescent cells, but underlying mechanisms of other potential senolytics are still not fully understood. Senolytics appear to have enormous potential across numerous medical fields in which the lasting persistence of senescent cells contributes to disease, especially as cancer therapeutics and in the arena of age-related diseases.

WO 2016/118014 A2 describes an anti-senescent peptide and methods for the use of this peptide in the treatment of age-related disorders.

It is an object of the present invention to provide a novel use for already known substances as well as to provide novel substances that are suited to be used as senolytic.

This object is achieved with the novel medical use (i.e., in-vivo use) of a compound according to claim 1 as senolytic. Such a compound has a chemical structure according to general formula (I), according to any of general formulae (II), (III), or (IV), or is a pharmaceutically acceptable salt or hydrate thereof:

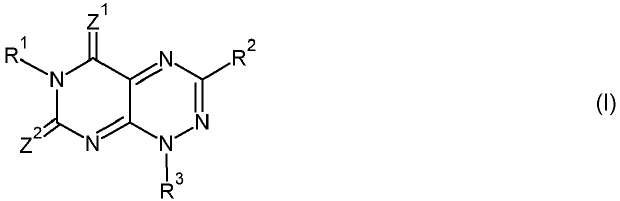

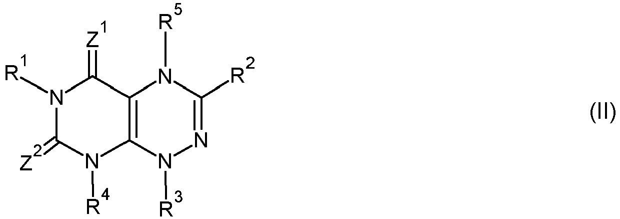

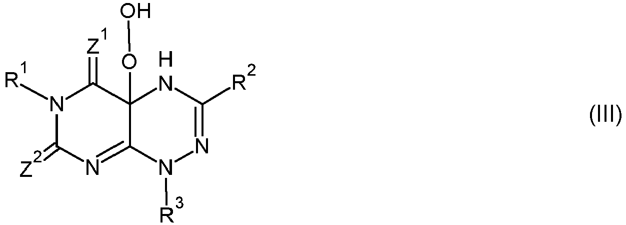

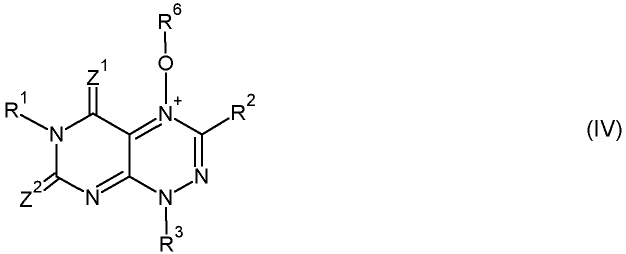

In this context, the residues have the following meanings:
- R¹ =: H, C₁-C₁₀ alkyl (in particular C₂-C₉ alkyl, in particular C₃-C₈ alkyl, in particular C₄-C₇ alkyl, in particular C₅-C₆ alkyl), C₃-C₂₀ cycloalkyl (in particular C₄-C₁₉ cycloalkyl, in particular C₅-C₁₈ cycloalkyl, in particular C₆-C₁₇ cycloalkyl, in particular C₇-C₁₆ cycloalkyl, in particular C₈-C₁₅ cycloalkyl, in particular C₉-C₁₄ cycloalkyl, in particular C₁₀-C₁₃ cycloalkyl, in particular C₁₁-C₁₂ cycloalkyl), heteroatom-substituted C₃-C₂₀ cycloalkyl (in particular heteroatom-substituted C₄-C₁₉ cycloalkyl, in particular heteroatom-substituted C₅-C₁₈ cycloalkyl, in particular heteroatom-substituted C₆-C₁₇ cycloalkyl, in particular heteroatom-substituted C₇-C₁₆ cycloalkyl, in particular heteroatom-substituted C₈-C₁₅ cycloalkyl, in particular heteroatom-substituted C₉-C₁₄ cycloalkyl, in particular heteroatom-substituted C₁₀-C₁₃ cycloalkyl, in particular heteroatom-substituted C₁₁-C₁₂ cycloalkyl), CF₃, CF₂H, CFH₂, optionally substituted piperidine, C₁-C₁₀ alkyl (in particular C₂-C₉ alkyl, in particular C₃-C₈ alkyl, in particular C₄-C₇ alkyl, in particular C₅-C₆ alkyl) carrying a primary, secondary or tertiary amino substituent, OH, CH₂-O-galactosyl, optionally substituted C₆-C₂₀ aryl (in particular optionally substituted C₇-C₁₉ aryl, in particular optionally substituted C₈-C₁₈ aryl, in particular optionally substituted C₉-C₁₇ aryl, in particular optionally substituted C₁₀-C₁₆ aryl, in particular optionally substituted C₁₁-C₁₅ aryl, in particular optionally substituted C₁₂-C₁₄ aryl), or optionally substituted C₅-C₂₀ heteroaryl (in particular optionally substituted C₆-C₁₉ heteroaryl, in particular optionally substituted C₇-C₁₈ heteroaryl, in particular optionally substituted C₈-C₁₇ heteroaryl, in particular optionally substituted C₉-C₁₆ heteroaryl, in particular optionally substituted C₁₀-C₁₅ heteroaryl, in particular optionally substituted C₁₁-C₁₄ heteroaryl, in particular optionally substituted C₁₂-C₁₃ heteroaryl),
- R² =: optionally substituted C₁-C₁₀ alkyl (in particular optionally substituted C₂-C₉ alkyl, in particular optionally substituted C₃-C₈ alkyl, in particular optionally substituted C₄-C₇ alkyl, in particular optionally substituted C₅-C₆ alkyl), optionally substituted C₇-C₃₀ alkyl aryl (in particular optionally substituted C₈-C₂₉ alkyl aryl, in particular optionally substituted C₉-C₂₈ alkyl aryl, in particular optionally substituted C₁₀-C₂₇ alkyl aryl, in particular optionally substituted C₁₁-C₂₆ alkyl aryl, in particular optionally substituted C₁₂-C₂₅ alkyl aryl, in particular optionally substituted C₁₃-C₂₄ alkyl aryl, in particular optionally substituted C₁₄-C₂₃ alkyl aryl, in particular optionally substituted C₁₅-C₂₂ alkyl aryl, in particular optionally substituted C₁₆-C₂₁ alkyl aryl, in particular optionally substituted C₁₇-C₂₀ alkyl aryl, in particular optionally substituted C₁₈-C₁₉ alkyl aryl), optionally substituted C₆-C₃₀ alkyl heteroaryl (in particular optionally substituted C₇-C₂₉ alkyl heteroaryl, in particular optionally substituted C₈-C₂₈ alkyl heteroaryl, in particular optionally substituted C₉-C₂₇ alkyl heteroaryl, in particular optionally substituted C₁₀-C₂₆ alkyl heteroaryl, in particular optionally substituted C₁₁-C₂₅ alkyl heteroaryl, in particular optionally substituted C₁₂-C₂₄ alkyl heteroaryl, in particular optionally substituted C₁₃-C₂₃ alkyl heteroaryl, in particular optionally substituted C₁₄-C₂₂ alkyl heteroaryl, in particular optionally substituted C₁₅-C₂₁ alkyl heteroaryl, in particular optionally substituted C₁₆-C₂₀ alkyl heteroaryl, in particular optionally substituted C₁₇-C₁₉ alkyl heteroaryl), optionally substituted C₆-C₂₀ aryl (in particular optionally substituted C₇-C₁₉ aryl, in particular optionally substituted C₈-C₁₈ aryl, in particular optionally substituted C₉-C₁₇ aryl, in particular optionally substituted C₁₀-C₁₆ aryl, in particular optionally substituted C₁₁-C₁₅ aryl, in particular optionally substituted C₁₂-C₁₄ aryl), optionally substituted C₆-C₂₀ aryl nitro (in particular optionally substituted C₇-C₁₉ aryl nitro, in particular optionally substituted C₈-C₁₈ aryl nitro, in particular optionally substituted C₉-C₁₇ aryl nitro, in particular optionally substituted C₁₀-C₁₆ aryl nitro, in particular optionally substituted C₁₁-C₁₅ aryl nitro, in particular optionally substituted C₁₂-C₁₄ aryl nitro), optionally substituted C₂-C₁₀ alkenyl (in particular optionally substituted C₂-C₉ alkenyl, in particular optionally substituted C₃-C₈ alkenyl, in particular optionally substituted C₄-C₇ alkenyl, in particular optionally substituted C₅-C₆ alkenyl), optionally substituted C₈-C₃₀ alkenyl aryl (in particular optionally substituted C₈-C₂₉ alkenyl aryl, in particular optionally substituted C₉-C₂₈ alkenyl aryl, in particular optionally substituted C₁₀-C₂₇ alkenyl aryl, in particular optionally substituted C₁₁-C₂₆ alkenyl aryl, in particular optionally substituted C₁₂-C₂₅ alkenyl aryl, in particular optionally substituted C₁₃-C₂₄ alkenyl aryl, in particular optionally substituted C₁₄-C₂₃ alkenyl aryl, in particular optionally substituted C₁₅-C₂₂ alkenyl aryl, in particular optionally substituted C₁₆-C₂₁ alkenyl aryl, in particular optionally substituted C₁₇-C₂₀ alkenyl aryl, in particular optionally substituted C₁₈-C₁₉ alkenyl aryl), optionally substituted C₈-C₃₀ alkenyl aryl nitro (in particular optionally substituted C₈-C₂₉ alkenyl aryl nitro, in particular optionally substituted C₉-C₂₈ alkenyl aryl nitro, in particular optionally substituted C₁₀-C₂₇ alkenyl aryl nitro, in particular optionally substituted C₁₁-C₂₆ alkenyl aryl nitro, in particular optionally substituted C₁₂-C₂₅ alkenyl aryl nitro, in particular optionally substituted C₁₃-C₂₄ alkenyl aryl nitro, in particular optionally substituted C₁₄-C₂₃ alkenyl aryl nitro, in particular optionally substituted C₁₅-C₂₂ alkenyl aryl nitro, in particular optionally substituted C₁₆-C₂₁ alkenyl aryl nitro, in particular optionally substituted C₁₇-C₂₀ alkenyl aryl nitro, in particular optionally substituted C₁₈-C₁₉ alkenyl aryl nitro), optionally substituted C₇-C₃₀ alkenyl heteroaryl (in particular optionally substituted C₈-C₂₉ alkenyl heteroaryl, in particular optionally substituted C₉-C₂₈ alkenyl heteroaryl, in particular optionally substituted C₁₀-C₂₇ alkenyl heteroaryl, in particular optionally substituted C₁₁-C₂₆ alkenyl heteroaryl, in particular optionally substituted C₁₂-C₂₅ alkenyl heteroaryl, in particular optionally substituted C₁₃-C₂₄ alkenyl heteroaryl, in particular optionally substituted C₁₄-C₂₃ alkenyl heteroaryl, in particular optionally substituted C₁₅-C₂₂ alkenyl heteroaryl, in particular optionally substituted C₁₆-C₂₁ alkenyl heteroaryl, in particular optionally substituted C₁₇-C₂₀ alkenyl heteroaryl, in particular optionally substituted C₁₈-C₁₉ alkenyl heteroaryl), optionally substituted C₂-C₁₀ alkinyl (in particular optionally substituted C₂-C₉ alkinyl, in particular optionally substituted C₃-C₈ alkinyl, in particular optionally substituted C₄-C₇ alkinyl, in particular optionally substituted C₅-C₆ alkinyl), optionally substituted C₈-C₃₀ alkinyl aryl (in particular optionally substituted C₈-C₂₉ alkinyl aryl, in particular optionally substituted C₉-C₂₈ alkinyl aryl, in particular optionally substituted C₁₀-C₂₇ alkinyl aryl, in particular optionally substituted C₁₁-C₂₆ alkinyl aryl, in particular optionally substituted C₁₂-C₂₅ alkinyl aryl, in particular optionally substituted C₁₃-C₂₄ alkinyl aryl, in particular optionally substituted C₁₄-C₂₃ alkinyl aryl, in particular optionally substituted C₁₅-C₂₂ alkinyl aryl, in particular optionally substituted C₁₆-C₂₁ alkinyl aryl, in particular optionally substituted C₁₇-C₂₀ alkinyl aryl, in particular optionally substituted C₁₈-C₁₉ alkinyl aryl), optionally substituted C₇-C₃₀ alkinyl heteroaryl (in particular optionally substituted C₈-C₂₉ alkinyl heteroaryl, in particular optionally substituted C₉-C₂₈ alkinyl heteroaryl, in particular optionally substituted C₁₀-C₂₇ alkinyl heteroaryl, in particular optionally substituted C₁₁-C₂₆ alkinyl heteroaryl, in particular optionally substituted C₁₂-C₂₅ alkinyl heteroaryl, in particular optionally substituted C₁₃-C₂₄ alkinyl heteroaryl, in particular optionally substituted C₁₄-C₂₃ alkinyl heteroaryl, in particular optionally substituted C₁₅-C₂₂ alkinyl heteroaryl, in particular optionally substituted C₁₆-C₂₁ alkinyl heteroaryl, in particular optionally substituted C₁₇-C₂₀ alkinyl heteroaryl, in particular optionally substituted C₁₈-C₁₉ alkinyl heteroaryl), optionally substituted C₁₁-C₃₀ heteroaryl aryl (in particular optionally substituted C₁₂-C₂₉ heteroaryl aryl, in particular optionally substituted C₁₃-C₂₈ heteroaryl aryl, in particular optionally substituted C₁₄-C₂₇ heteroaryl aryl, in particular optionally substituted C₁₅-C₂₆ heteroaryl aryl, in particular optionally substituted C₁₆-C₂₅ heteroaryl aryl, in particular optionally substituted C₁₇-C₂₄ heteroaryl aryl, in particular optionally substituted C₁₈-C₂₃ heteroaryl aryl, in particular optionally substituted C₁₉-C₂₂ heteroaryl aryl, in particular optionally substituted C₂₀-C₂₁ heteroaryl aryl), optionally substituted C₉-C₃₀ cyclyl aryl (in particular optionally substituted C₁₀-C₂₉ cyclyl aryl, in particular optionally substituted C₁₁-C₂₈ cyclyl aryl, in particular optionally substituted C₁₂-C₂₇ cyclyl aryl, in particular optionally substituted C₁₃-C₂₆ cyclyl aryl, in particular optionally substituted C₁₄-C₂₅ cyclyl aryl, in particular optionally substituted C₁₅-C₂₄ cyclyl aryl, in particular optionally substituted C₁₆-C₂₃ cyclyl aryl, in particular optionally substituted C₁₇-C₂₂ cyclyl aryl, in particular optionally substituted C₁₈-C₂₁ cyclyl aryl, in particular optionally substituted C₁₉-C₂₀ cyclyl aryl), optionally substituted C₈-C₃₀ heterocyclyl aryl (in particular optionally substituted C₉-C₂₉ heterocyclyl aryl, in particular optionally substituted C₁₀-C₂₉ heterocyclyl aryl, in particular optionally substituted C₁₁-C₂₈ heterocyclyl aryl, in particular optionally substituted C₁₂-C₂₇ heterocyclyl aryl, in particular optionally substituted C₁₃-C₂₆ heterocyclyl aryl, in particular optionally substituted C₁₄-C₂₅ heterocyclyl aryl, in particular optionally substituted C₁₅-C₂₄ heterocyclyl aryl, in particular optionally substituted C₁₆-C₂₃ heterocyclyl aryl, in particular optionally substituted C₁₇-C₂₂ heterocyclyl aryl, in particular optionally substituted C₁₈-C₂₁ heterocyclyl aryl, in particular optionally substituted C₁₉-C₂₀ heterocyclyl aryl), optionally substituted C₃-C₂₀ cycloalkyl (in particular optionally substituted C₄-C₁₉ cycloalkyl, in particular optionally substituted C₅-C₁₈ cycloalkyl, in particular optionally substituted C₆-C₁₇ cycloalkyl, in particular optionally substituted C₇-C₁₆ cycloalkyl, in particular optionally substituted C₈-C₁₅ cycloalkyl, in particular optionally substituted C₉-C₁₄ cycloalkyl, in particular optionally substituted C₁₀-C₁₃ cycloalkyl, in particular optionally substituted C₁₁-C₁₂ cycloalkyl), halogen, OH, C₁-C₁₀ alkoxy (in particular C₂-C₉ alkoxy, in particular C₃-C₈ alkoxy, in particular C₄-C₇ alkoxy, in particular C₅-C₆ alkoxy), C₆-C₂₀ aryloxy (in particular C₇-C₁₉ aryloxy, in particular C₈-C₁₈ aryloxy, in particular C₉-C₁₇ aryloxy, in particular C₁₀-C₁₆ aryloxy, in particular C₁₁-C₁₅ aryloxy, in particular C₁₂-C₁₄ aryloxy), amino, substituted amino, CN, carboxy, optionally substituted C₁-C₁₀ alkyl carboxy (in particular optionally substituted C₂-C₉ alkyl carboxy, in particular optionally substituted C₃-C₈ alkyl carboxy, in particular optionally substituted C₄-C₇ alkyl carboxy, in particular optionally substituted C₅-C₆ alkyl carboxy), optionally substituted C₆-C₂₀ aryl carboxy (in particular optionally substituted C₇-C₁₉ aryl carboxy, in particular optionally substituted C₈-C₁₈ aryl carboxy, in particular optionally substituted C₉-C₁₇ aryl carboxy, in particular optionally substituted C₁₀-C₁₆ aryl carboxy, in particular optionally substituted C₁₁-C₁₅ aryl carboxy, in particular optionally substituted C₁₂-C₁₄ aryl carboxy), carbamido, optionally substituted C₅-C₂₀ heteroaryl (in particular optionally substituted C₆-C₁₉ heteroaryl, in particular optionally substituted C₇-C₁₈ heteroaryl, in particular optionally substituted C₈-C₁₇ heteroaryl, in particular optionally substituted C₉-C₁₆ heteroaryl, in particular optionally substituted C₁₀-C₁₅ heteroaryl, in particular optionally substituted C₁₁-C₁₄ heteroaryl, in particular optionally substituted C₁₂-C₁₃ heteroaryl), optionally substituted C₂-C₂₀ heterocyclyl (in particular optionally substituted C₃-C₁₉ heterocyclyl, in particular optionally substituted C₄-C₁₉ heterocyclyl, in particular optionally substituted C₅-C₁₈ heterocyclyl, in particular optionally substituted C₆-C₁₇ heterocyclyl, in particular optionally substituted C₇-C₁₆ heterocyclyl, in particular optionally substituted C₈-C₁₅ heterocyclyl, in particular optionally substituted C₉-C₁₄ heterocyclyl, in particular optionally substituted C₁₀-C₁₃ heterocyclyl, in particular optionally substituted C₁₁-C₁₂ heterocyclyl), heteroatom-substituted C₆-C₂₀ cycloalkyl (in particular heteroatom-substituted C₇-C₁₉ cycloalkyl, in particular heteroatom-substituted C₈-C₁₈ cycloalkyl, in particular heteroatom-substituted C₉-C₁₇ cycloalkyl, in particular heteroatom-substituted C₁₀-C₁₆ cycloalkyl, in particular heteroatom-substituted C₁₁-C₁₅ cycloalkyl, in particular heteroatom-substituted C₁₂-C₁₄ cycloalkyl, in particular heteroatom-substituted C₁₃-C₁₄ cycloalkyl), optionally substituted SH, sulfonamido, or sulfone,
- R³ =: C₁-C₁₀ alkyl (in particular C₂-C₉ alkyl, in particular C₃-C₈ alkyl, in particular C₄-C₇ alkyl, in particular C₅-C₆ alkyl), C₃-C₂₀ cycloalkyl (in particular C₄-C₁₉ cycloalkyl, in particular C₅-C₁₈ cycloalkyl, in particular C₆-C₁₇ cycloalkyl, in particular C₇-C₁₆ cycloalkyl, in particular C₈-C₁₅ cycloalkyl, in particular C₉-C₁₄ cycloalkyl, in particular C₁₀-C₁₃ cycloalkyl, in particular C₁₁-C₁₂ cycloalkyl), heteroatom-substituted C₃-C₂₀ cycloalkyl (in particular heteroatom-substituted C₄-C₁₉ cycloalkyl, in particular heteroatom-substituted C₅-C₁₈ cycloalkyl, in particular heteroatom-substituted C₆-C₁₇ cycloalkyl, in particular heteroatom-substituted C₇-C₁₆ cycloalkyl, in particular heteroatom-substituted C₈-C₁₅ cycloalkyl, in particular heteroatom-substituted C₉-C₁₄ cycloalkyl, in particular heteroatom-substituted C₁₀-C₁₃ cycloalkyl, in particular heteroatom-substituted C₁₁-C₁₂ cycloalkyl), CF₃, CF₂H, CFH₂, optionally substituted piperidine, C₁-C₁₀ alkyl (in particular C₂-C₉ alkyl, in particular C₃-C₈ alkyl, in particular C₄-C₇ alkyl, in particular C₅-C₆ alkyl) carrying a primary, secondary or tertiary amino substituent, OH, CH₂-O-galactosyl, optionally substituted C₆-C₂₀ aryl (in particular optionally substituted C₇-C₁₉ aryl, in particular optionally substituted C₈-C₁₈ aryl, in particular optionally substituted C₉-C₁₇ aryl, in particular optionally substituted C₁₀-C₁₆ aryl, in particular optionally substituted C₁₁-C₁₅ aryl, in particular optionally substituted C₁₂-C₁₄ aryl), or optionally substituted C₅-C₂₀ heteroaryl (in particular optionally substituted C₆-C₁₉ heteroaryl, in particular optionally substituted C₇-C₁₈ heteroaryl, in particular optionally substituted C₈-C₁₇ heteroaryl, in particular optionally substituted C₉-C₁₆ heteroaryl, in particular optionally substituted C₁₀-C₁₅ heteroaryl, in particular optionally substituted C₁₁-C₁₄ heteroaryl, in particular optionally substituted C₁₂-C₁₃ heteroaryl),
- R⁴, R⁵ =: independently from each other H, C₁-C₁₀ alkyl (in particular C₂-C₉ alkyl, in particular C₃-C₈ alkyl, in particular C₄-C₇ alkyl, in particular C₅-C₆ alkyl), C₃-C₂₀ cycloalkyl (in particular C₄-C₁₉ cycloalkyl, in particular C₅-C₁₈ cycloalkyl, in particular C₆-C₁₇ cycloalkyl, in particular C₇-C₁₆ cycloalkyl, in particular C₈-C₁₅ cycloalkyl, in particular C₉-C₁₄ cycloalkyl, in particular C₁₀-C₁₃ cycloalkyl, in particular C₁₁-C₁₂ cycloalkyl), heteroatom-substituted C₃-C₂₀ cycloalkyl (in particular heteroatom-substituted C₄-C₁₉ cycloalkyl, in particular heteroatom-substituted C₅-C₁₈ cycloalkyl, in particular heteroatom-substituted C₆-C₁₇ cycloalkyl, in particular heteroatom-substituted C₇-C₁₆ cycloalkyl, in particular heteroatom-substituted C₈-C₁₅ cycloalkyl, in particular heteroatom-substituted C₉-C₁₄ cycloalkyl, in particular heteroatom-substituted C₁₀-C₁₃ cycloalkyl, in particular heteroatom-substituted C₁₁-C₁₂ cycloalkyl), CF₃, CF₂H, CFH₂, optionally substituted piperidine, C₁-C₁₀ alkyl (in particular C₂-C₉ alkyl, in particular C₃-C₈ alkyl, in particular C₄-C₇ alkyl, in particular C₅-C₆ alkyl)carrying a primary, secondary or tertiary amino substituent, OH, CH₂-O-galactosyl, optionally substituted C₆-C₂₀ aryl (in particular optionally substituted C₇-C₁₉ aryl, in particular optionally substituted C₈-C₁₈ aryl, in particular optionally substituted C₉-C₁₇ aryl, in particular optionally substituted C₁₀-C₁₆ aryl, in particular optionally substituted C₁₁-C₁₅ aryl, in particular optionally substituted C₁₂-C₁₄ aryl), or optionally substituted C₅-C₂₀ heteroaryl (in particular optionally substituted C₆-C₁₉ heteroaryl, in particular optionally substituted C₇-C₁₈ heteroaryl, in particular optionally substituted C₈-C₁₇ heteroaryl, in particular optionally substituted C₉-C₁₆ heteroaryl, in particular optionally substituted C₁₀-C₁₅ heteroaryl, in particular optionally substituted C₁₁-C₁₄ heteroaryl, in particular optionally substituted C₁₂-C₁₃ heteroaryl), or a protecting group chosen from i) *tert*-butyloxycarbonyl, ii) trityl, iii) acetyl, iv) C₁-C₁₀ acyl (in particular C₂-C₉ acyl, in particular C₃-C₈ acyl, in particular C₄-C₇ acyl, in particular C₅-C₆ acyl) residues, and v) monosubstituted or disubstituted compounds according to general formula (II), wherein R⁴ and R⁵ denote H,
- R⁶ =: absent (i.e., the oxygen atom carries a negative charge) or a protecting group chosen from C₁-C₁₀ acyl (in particular C₂-C₉ acyl, in particular C₃-C₈ acyl, in particular C₄-C₇ acyl, in particular C₅-C₆ acyl), acetyl, ester residues, benzoyl, benzyl, trityl, and ether residues, and
- Z¹, Z² =: independently from each other O or S.

In an embodiment, residues R⁴ and R⁵ denote hydrogen. Then, the compounds according to general formula (II) represent the reduced form of the compounds according to general formula (I). They can also be denoted as reduced derivative of the compounds according to general formula (I).

The compounds according to general formula (III) represent the hydroperoxy derivative of the compounds according to general formula (I).

The compounds according to general formula (IV) represent the N-oxide derivatives of the compounds according to general formula (I).

Thus, the compounds according to general formula (I), (II), (III), and (IV) are closely interrelated. As will be explained below in more detail, they represent different forms of one and the same molecule during a redox cycle that is passed through by the molecule within a senescent cell to exhibit its senolytic activity.

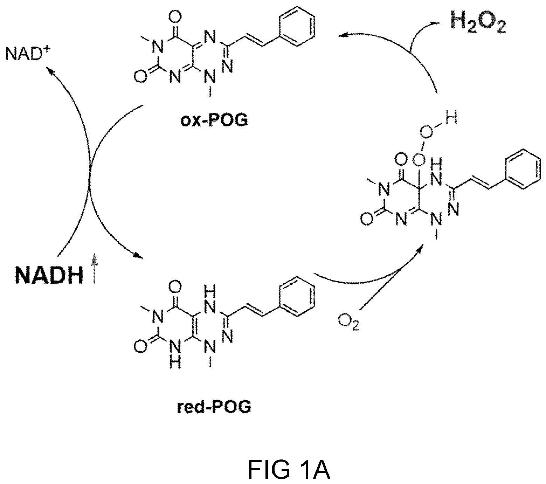

The compounds according to general formula (I) are able to produce hydrogen peroxide (H₂O₂, HP) via a redox mechanism. Therefore, it will be referred to them in the following also as "peroxygenins" (POG). In the presence of a suitable biochemical reducing agent such as dithiothreitol (DTT), reduced nicotinamide adenine dinucleotide (NADH), reduced nicotinamide adenine dinucleotide phosphate (NADPH), or dihydrolipoic acid (DHLA), the oxidized form ox-POG can be reduced to the reduced form red-POG, which then forms a hydroperoxy species with molecular oxygen (confer Figure 1A). The original form of POG is recovered by release of HP, which means that the formation of HP occurs in a catalytic cycle driven by a reducing agent. POG activation is promoted by senescent cells, because senescent cells are metabolically altered compared to healthy cells and specifically exhibit higher glucose consumption and higher oxidative stress (reactive oxygen species, ROS, like HP). Indeed, the inventors found that cells undergoing senescence generate significantly higher cellular levels of the reducing equivalent NADH. Importantly, glutathione (GSH) levels were found to be depleted in senescent cells; accordingly, GSSG/GSH ratios were strongly increased due to oxidative stress.

In consequence, POG are efficiently reduced by NADH in senescent cells, generating high concentrations of HP, which are only inefficiently detoxified by the already reduced GSH levels, thereby selectively killing senescent cells (confer Figure 1A). Such mode of activation applies to all types of senescence, thus, conceptually constituting POG as a novel pan-senolytic drug.

In an embodiment, Z¹ is O and Z² is S. In an embodiment, Z¹ is S and Z² is O. In an embodiment, Z¹ is O and Z² is O. In an embodiment, Z¹ is S and Z² is S.

Generally, the nitro group is a particularly appropriate substituent of all hydrocarbon systems (such as alkyl and alkenyl chains, cycloalkyl, heterocyclyl, aryl, and heteroaryl groups) of the various residues, in particular of residue R². In an embodiment, the nitro group serves as substituent of any of the hydrocarbon systems of the various residues, in particular of residue R². In case of an optionally substituted aryl nitro (such as an optionally substituted nitrostyryl) or alkenyl aryl nitro (such as an optionally substituted alkenyl nitrostyryl), a further substituent (besides the already present nitro group) can be another nitro group or a different substituent.

In an embodiment, residue R² is a residue according to any of the following formulae, wherein the dashed line indicates a bond by which the residue R² is covalently bound to the triazine ring of the compound according to general formulae (I), (II), (III), or (IV):

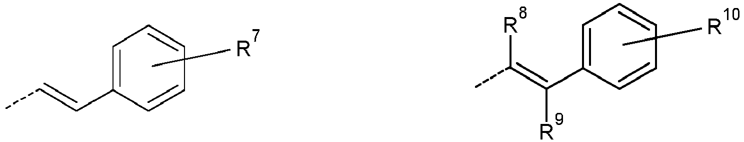

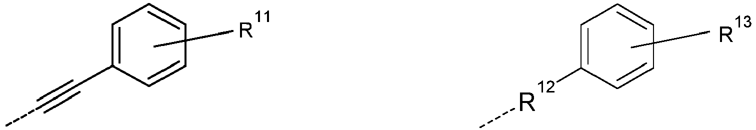

In this context, the residues have the following meanings:
- R⁷, R¹⁰, R¹¹, R¹³ =: independently from each other H, NO₂, optionally substituted C₁-C₁₀ alkyl (in particular optionally substituted C₂-C₉ alkyl, in particular optionally substituted C₃-C₈ alkyl, in particular optionally substituted C₄-C₇ alkyl, in particular optionally substituted C₅-C₆ alkyl), optionally substituted C₂-C₁₀ alkenyl (in particular optionally substituted C₂-C₉ alkenyl, in particular optionally substituted C₃-C₈ alkenyl, in particular optionally substituted C₄-C₇ alkenyl, in particular optionally substituted C₅-C₆ alkenyl), optionally substituted C₃-C₂₀ cycloalkyl (in particular optionally substituted C₄-C₁₉ cycloalkyl, in particular optionally substituted C₅-C₁₈ cycloalkyl, in particular optionally substituted C₆-C₁₇ cycloalkyl, in particular optionally substituted C₇-C₁₆ cycloalkyl, in particular optionally substituted C₈-C₁₅ cycloalkyl, in particular optionally substituted C₉-C₁₄ cycloalkyl, in particular optionally substituted C₁₀-C₁₃ cycloalkyl, in particular optionally substituted C₁₁-C₁₂ cycloalkyl), heteroatom-substituted C₃-C₂₀ cycloalkyl (in particular heteroatom-substituted C₄-C₁₉ cycloalkyl, in particular heteroatom-substituted C₅-C₁₈ cycloalkyl, in particular heteroatom-substituted C₆-C₁₇ cycloalkyl, in particular heteroatom-substituted C₇-C₁₆ cycloalkyl, in particular heteroatom-substituted C₈-C₁₅ cycloalkyl, in particular heteroatom-substituted C₉-C₁₄ cycloalkyl, in particular heteroatom-substituted C₁₀-C₁₃ cycloalkyl, in particular heteroatom-substituted C₁₁-C₁₂ cycloalkyl), halogen, OH, C₁-C₁₀ alkoxy (in particular C₂-C₉ alkoxy, in particular C₃-C₈ alkoxy, in particular C₄-C₇ alkoxy, in particular C₅-C₆ alkoxy), C₆-C₂₀ aryloxy (in particular C₇-C₁₉ aryloxy, in particular C₈-C₁₈ aryloxy, in particular C₉-C₁₇ aryloxy, in particular C₁₀-C₁₆ aryloxy, in particular C₁₁-C₁₅ aryloxy, in particular C₁₂-C₁₄ aryloxy), amino, substituted amino, CN, carboxy, optionally substituted C₁-C₁₀ alkyl carboxy (in particular optionally substituted C₂-C₉ alkyl carboxy, in particular optionally substituted C₃-C₈ alkyl carboxy, in particular optionally substituted C₄-C₇ alkyl carboxy, in particular optionally substituted C₅-C₆ alkyl carboxy), optionally substituted C₆-C₂₀ aryl-carboxy (in particular optionally substituted C₇-C₁₉ aryl carboxy, in particular optionally substituted C₈-C₁₈ aryl carboxy, in particular optionally substituted C₉-C₁₇ aryl carboxy, in particular optionally substituted C₁₀-C₁₆ aryl carboxy, in particular optionally substituted C₁₁-C₁₅ aryl carboxy, in particular optionally substituted C₁₂-C₁₄ aryl carboxy), carbamido, optionally substituted C₅-C₂₀ heteroaryl (in particular optionally substituted C₆-C₁₉ heteroaryl, in particular optionally substituted C₇-C₁₈ heteroaryl, in particular optionally substituted C₈-C₁₇ heteroaryl, in particular optionally substituted C₉-C₁₆ heteroaryl, in particular optionally substituted C₁₀-C₁₅ heteroaryl, in particular optionally substituted C₁₁-C₁₄ heteroaryl, in particular optionally substituted C₁₂-C₁₃ heteroaryl), optionally substituted C₂-C₂₀ heterocyclyl (in particular optionally substituted C₃-C₁₉ heterocyclyl, in particular optionally substituted C₄-C₁₉ heterocyclyl, in particular optionally substituted C₅-C₁₈ heterocyclyl, in particular optionally substituted C₆-C₁₇ heterocyclyl, in particular optionally substituted C₇-C₁₆ heterocyclyl, in particular optionally substituted C₈-C₁₅ heterocyclyl, in particular optionally substituted C₉-C₁₄ heterocyclyl, in particular optionally substituted C₁₀-C₁₃ heterocyclyl, in particular optionally substituted C₁₁-C₁₂ heterocyclyl), optionally substituted C₆-C₂₀ aryl (in particular optionally substituted C₇-C₁₉ aryl, in particular optionally substituted C₈-C₁₈ aryl, in particular optionally substituted C₉-C₁₇ aryl, in particular optionally substituted C₁₀-C₁₆ aryl, in particular optionally substituted C₁₁-C₁₅ aryl, in particular optionally substituted C₁₂-C₁₄ aryl), optionally substituted SH, sulfonamido, or sulfone,
- R⁸, R⁹=: independently from each other H, optionally substituted C₁-C₁₀ alkyl (in particular optionally substituted C₂-C₉ alkyl, in particular optionally substituted C₃-C₈ alkyl, in particular optionally substituted C₄-C₇ alkyl, in particular optionally substituted C₅-C₆ alkyl), optionally substituted C₂-C₁₀ alkenyl (in particular optionally substituted C₂-C₉ alkenyl, in particular optionally substituted C₃-C₈ alkenyl, in particular optionally substituted C₄-C₇ alkenyl, in particular optionally substituted C₅-C₆ alkenyl), optionally substituted C₃-C₂₀ cycloalkyl (in particular optionally substituted C₄-C₁₉ cycloalkyl, in particular optionally substituted C₅-C₁₈ cycloalkyl, in particular optionally substituted C₆-C₁₇ cycloalkyl, in particular optionally substituted C₇-C₁₆ cycloalkyl, in particular optionally substituted C₈-C₁₅ cycloalkyl, in particular optionally substituted C₉-C₁₄ cycloalkyl, in particular optionally substituted C₁₀-C₁₃ cycloalkyl, in particular optionally substituted C₁₁-C₁₂ cycloalkyl), heteroatom-substituted C₃-C₂₀ cycloalkyl (in particular heteroatom-substituted C₄-C₁₉ cycloalkyl, in particular heteroatom-substituted C₅-C₁₈ cycloalkyl, in particular heteroatom-substituted C₆-C₁₇ cycloalkyl, in particular heteroatom-substituted C₇-C₁₆ cycloalkyl, in particular heteroatom-substituted C₈-C₁₅ cycloalkyl, in particular heteroatom-substituted C₉-C₁₄ cycloalkyl, in particular heteroatom-substituted C₁₀-C₁₃ cycloalkyl, in particular heteroatom-substituted C₁₁-C₁₂ cycloalkyl), halogen, OH, C₁-C₁₀ alkoxy (in particular C₂-C₉ alkoxy, in particular C₃-C₈ alkoxy, in particular C₄-C₇ alkoxy, in particular C₅-C₆ alkoxy), C₆-C₂₀ aryloxy (in particular C₇-C₁₉ aryloxy, in particular C₈-C₁₈ aryloxy, in particular C₉-C₁₇ aryloxy, in particular C₁₀-C₁₆ aryloxy, in particular C₁₁-C₁₅ aryloxy, in particular C₁₂-C₁₄ aryloxy), amino, substituted amino, CN, carboxy, optionally substituted C₁-C₁₀ alkyl carboxy (in particular optionally substituted C₂-C₉ alkyl carboxy, in particular optionally substituted C₃-C₈ alkyl carboxy, in particular optionally substituted C₄-C₇ alkyl carboxy, in particular optionally substituted C₅-C₆ alkyl carboxy), optionally substituted C₆-C₂₀ aryl-carboxy (in particular optionally substituted C₇-C₁₉ aryl carboxy, in particular optionally substituted C₈-C₁₈ aryl carboxy, in particular optionally substituted C₉-C₁₇ aryl carboxy, in particular optionally substituted C₁₀-C₁₆ aryl carboxy, in particular optionally substituted C₁₁-C₁₅ aryl carboxy, in particular optionally substituted C₁₂-C₁₄ aryl carboxy), carbamido, optionally substituted C₅-C₂₀ heteroaryl (in particular optionally substituted C₆-C₁₉ heteroaryl, in particular optionally substituted C₇-C₁₈ heteroaryl, in particular optionally substituted C₈-C₁₇ heteroaryl, in particular optionally substituted C₉-C₁₆ heteroaryl, in particular optionally substituted C₁₀-C₁₅ heteroaryl, in particular optionally substituted C₁₁-C₁₄ heteroaryl, in particular optionally substituted C₁₂-C₁₃ heteroaryl), optionally substituted C₂-C₂₀ heterocyclyl (in particular optionally substituted C₃-C₁₉ heterocyclyl, in particular optionally substituted C₄-C₁₉ heterocyclyl, in particular optionally substituted C₅-C₁₈ heterocyclyl, in particular optionally substituted C₆-C₁₇ heterocyclyl, in particular optionally substituted C₇-C₁₆ heterocyclyl, in particular optionally substituted C₈-C₁₅ heterocyclyl, in particular optionally substituted C₉-C₁₄ heterocyclyl, in particular optionally substituted C₁₀-C₁₃ heterocyclyl, in particular optionally substituted C₁₁-C₁₂ heterocyclyl), optionally substituted C₆-C₂₀ aryl (in particular optionally substituted C₇-C₁₉ aryl, in particular optionally substituted C₈-C₁₈ aryl, in particular optionally substituted C₉-C₁₇ aryl, in particular optionally substituted C₁₀-C₁₆ aryl, in particular optionally substituted C₁₁-C₁₅ aryl, in particular optionally substituted C₁₂-C₁₄ aryl), optionally substituted SH, sulfonamido, or sulfone, CF₃, CF₂H, or CFH₂, and
- R¹² =: independently from each other optionally substituted C₁-C₁₀ alkyl (in particular optionally substituted C₂-C₉ alkyl, in particular optionally substituted C₃-C₈ alkyl, in particular optionally substituted C₄-C₇ alkyl, in particular optionally substituted C₅-C₆ alkyl), optionally substituted C₂-C₁₀ alkenyl (in particular optionally substituted C₂-C₉ alkenyl, in particular optionally substituted C₃-C₈ alkenyl, in particular optionally substituted C₄-C₇ alkenyl, in particular optionally substituted C₅-C₆ alkenyl), optionally substituted C₂-C₁₀ alkinyl (in particular optionally substituted C₂-C₉ alkinyl, in particular optionally substituted C₃-C₈ alkinyl, in particular optionally substituted C₄-C₇ alkinyl, in particular optionally substituted C₅-C₆ alkinyl), optionally substituted C₆-C₂₀ aryl (in particular optionally substituted C₇-C₁₉ aryl, in particular optionally substituted C₈-C₁₈ aryl, in particular optionally substituted C₉-C₁₇ aryl, in particular optionally substituted C₁₀-C₁₆ aryl, in particular optionally substituted C₁₁-C₁₅ aryl, in particular optionally substituted C₁₂-C₁₄ aryl), optionally substituted C₅-C₂₀ heteroaryl (in particular optionally substituted C₆-C₁₉ heteroaryl, in particular optionally substituted C₇-C₁₈ heteroaryl, in particular optionally substituted C₈-C₁₇ heteroaryl, in particular optionally substituted C₉-C₁₆ heteroaryl, in particular optionally substituted C₁₀-C₁₅ heteroaryl, in particular optionally substituted C₁₁-C₁₄ heteroaryl, in particular optionally substituted C₁₂-C₁₃ heteroaryl), optionally substituted C₃-C₂₀ cycloalkyl (in particular optionally substituted C₄-C₁₉ cycloalkyl, in particular optionally substituted C₅-C₁₈ cycloalkyl, in particular optionally substituted C₆-C₁₇ cycloalkyl, in particular optionally substituted C₇-C₁₆ cycloalkyl, in particular optionally substituted C₈-C₁₅ cycloalkyl, in particular optionally substituted C₉-C₁₄ cycloalkyl, in particular optionally substituted C₁₀-C₁₃ cycloalkyl, in particular optionally substituted C₁₁-C₁₂ cycloalkyl), optionally substituted C₃-C₂₀ cyclyl (in particular optionally substituted C₄-C₁₉ cyclyl, in particular optionally substituted C₅-C₁₈ cyclyl, in particular optionally substituted C₆-C₁₇ cyclyl, in particular optionally substituted C₇-C₁₆ cyclyl, in particular optionally substituted C₈-C₁₅ cyclyl, in particular optionally substituted C₉-C₁₄ cyclyl, in particular optionally substituted C₁₀-C₁₃ cyclyl, in particular optionally substituted C₁₁-C₁₂ cyclyl), or optionally substituted C₂-C₂₀ heterocyclyl (in particular optionally substituted C₃-C₁₉ heterocyclyl, in particular optionally substituted C₄-C₁₉ heterocyclyl, in particular optionally substituted C₅-C₁₈ heterocyclyl, in particular optionally substituted C₆-C₁₇ heterocyclyl, in particular optionally substituted C₇-C₁₆ heterocyclyl, in particular optionally substituted C₈-C₁₅ heterocyclyl, in particular optionally substituted C₉-C₁₄ heterocyclyl, in particular optionally substituted C₁₀-C₁₃ heterocyclyl, in particular optionally substituted C₁₁-C₁₂ heterocyclyl).

In an embodiment, residue R² is a residue according to any of the following formulae, wherein the dashed line indicates a bond by which the residue R² is covalently bound to the triazine ring of the compound according to general formulae (I), (II), (III), or (IV):

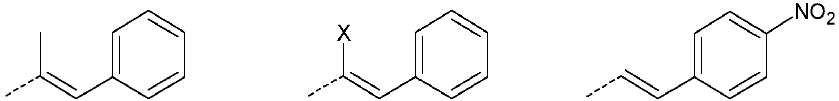

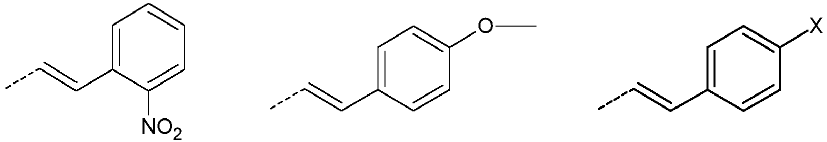

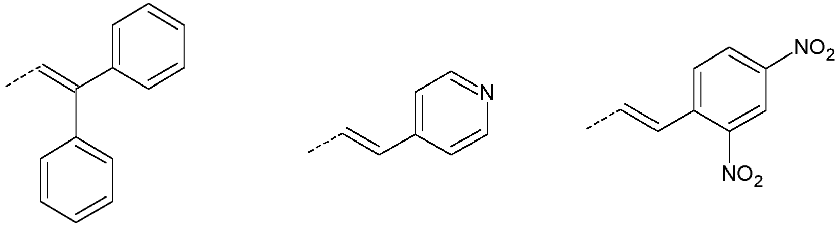

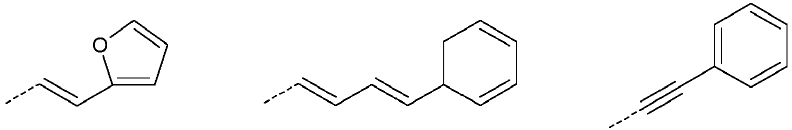

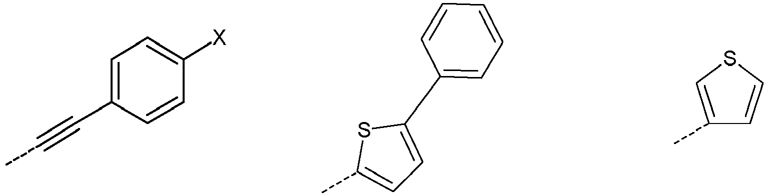

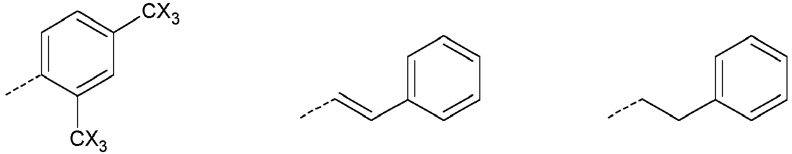

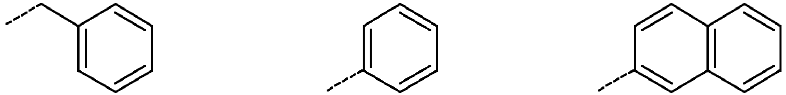

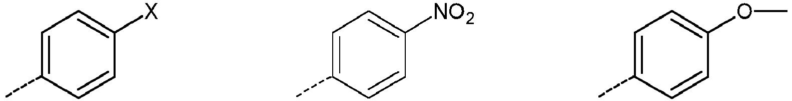

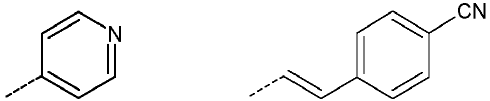

In this context, residue X has one of the following meanings: F, Cl, Br, or I, in particular F or Br, in particular F.

In an embodiment, residue R¹ is a methyl residue. In an embodiment, residue R¹ is a hydrogen atom.

In an embodiment, residue R³ is a methyl residue.

In an embodiment, residue R¹ is a methyl residue or a hydrogen atom and residue R³ is chosen from methyl, ethyl, isopropyl, phenyl, and substituted phenyl.

In an embodiment, residue R¹ is a methyl residue or a hydrogen atom and R³ is a methyl residue.

In an embodiment, the optionally substituted phenyl, in particular the optionally substituted phenyl of residue R² or residue R³, is a phenyl residue substituted with a nitro group or a cyano group.

Particular appropriate examples of compounds for the claimed use are listed in the following Table 1.

**Table 1: Particular appropriate examples of compounds for the claimed use.**

| **No.** | **Name** | **Structure** |
|---|---|---|
| 1 | alpha-methylstyryl POG | 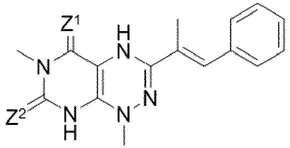 |
| 1.1 | red-alpha-methylstyryl POG | 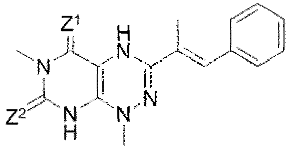 |
| 1.2 | hydroperoxy-alpha-methylstyryl POG | 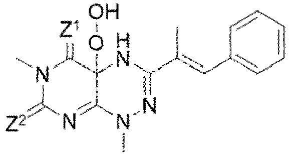 |
| 2 | alpha-bromostyryl POG | 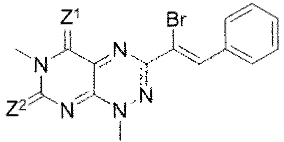 |
| 2.1 | red-alpha-bromostyryl POG | 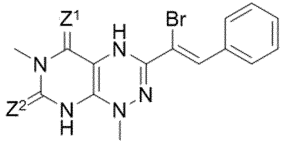 |
| 2.2 | hydroperoxy-alpha-bromostyryl POG | 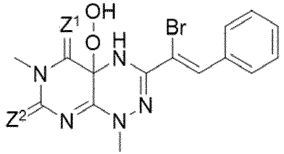 |
| 3 | p-nitrostyryl POG | 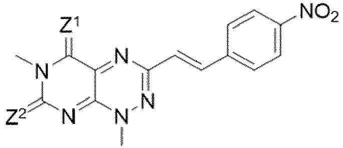 |
| 3.1 | red-p-nitrostyryl POG | 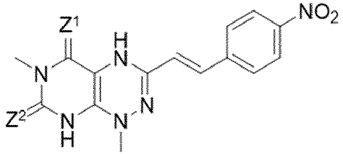 |
| 3.2 | hydroperoxy-p-nitrostyryl POG | 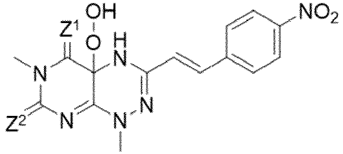 |
| 4 | o-nitrostyryl POG | 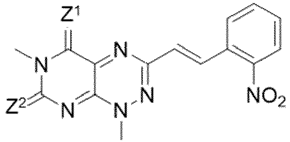 |
| 4.1 | red-o-nitrostyryl POG | 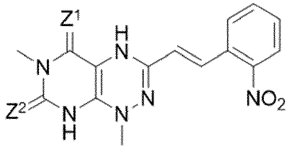 |
| 4.2 | hydroperoxy-o-nitrostyryl POG | 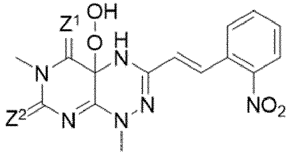 |
| 5 | p-methoxystyryl POG | 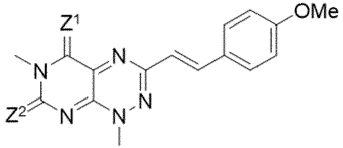 |
| 5.1 | red-p-methoxystyryl POG | 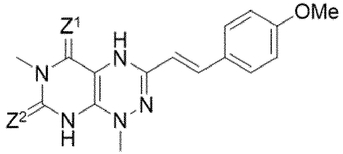 |
| 5.2 | hydroperoxy-p-methoxystyryl POG | 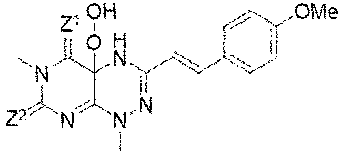 |
| 6 | p-fluorostyryl POG | 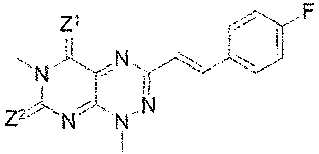 |
| 6.1 | red-p-fluorostyryl POG | 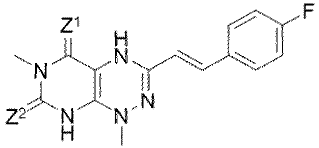 |
| 6.2 | hydroperoxy-p-fluorostyryl POG | 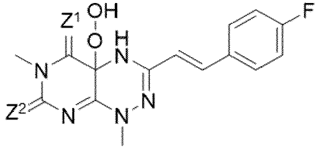 |
| 7 | diphenylstyryl POG | 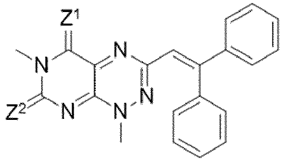 |
| 7.1 | red-diphenylstyryl POG | 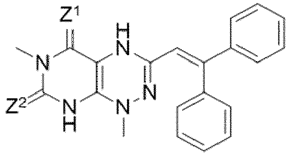 |
| 7.2 | hydroperoxy-diphenylstyryl POG | 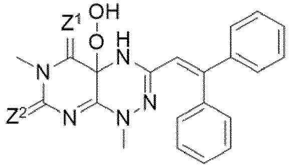 |
| 8 | 4-pyridinestyryl POG | 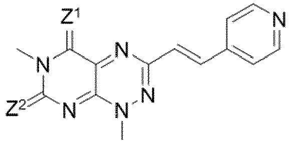 |
| 8.1 | red-4-pyridinestyryl POG | 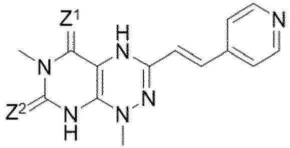 |
| 8.2 | hydroperoxy-4-pyridinestyryl POG | 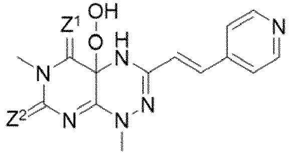 |
| 9 | dinitrostyryl POG | 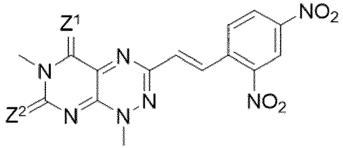 |
| 9.1 | red-dinitrostyryl POG | 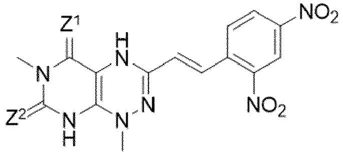 |
| 9.2 | hydroperxy-dinitrostyryl POG | 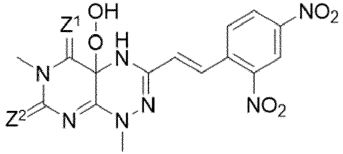 |
| 10 | furanacroleinstyryl POG | 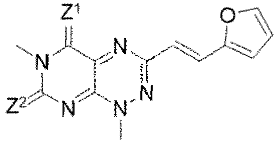 |
| 10.1 | red-furanacroleinstyryl POG | 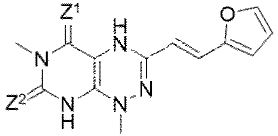 |
| 10.2 | hydroperoxy-furanacroleinstyryl POG | 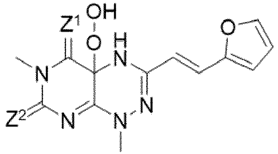 |
| 11 | 2m-styryl POG | 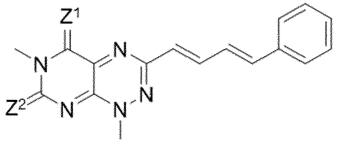 |
| 11.1 | red-2m-styryl POG | 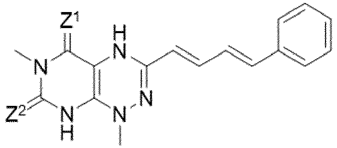 |
| 11.2 | hydroperoxy-2m-styryl POG | 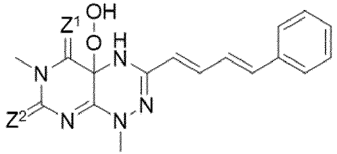 |
| 12 | ethine POG | 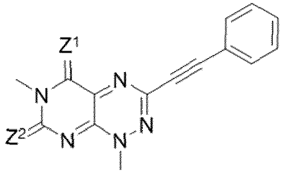 |
| 12.1 | red-ethine POG | 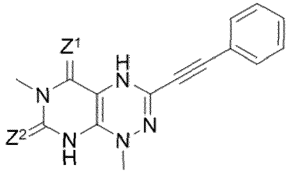 |
| 12.2 | hydroperoxy-ethine POG | 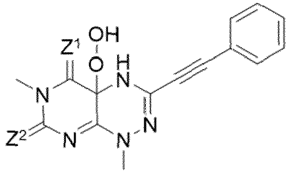 |
| 13 | p-fluoroethine POG | 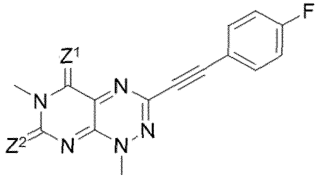 |
| 13.1 | red-p-fluoroethine POG | 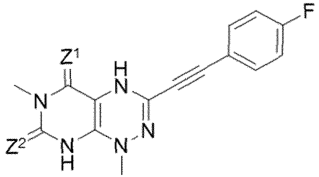 |
| 13.2 | hydroperoxy-p-fluoroethine POG | 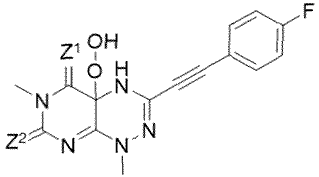 |
| 14 | phenyl-thiophenyl POG | 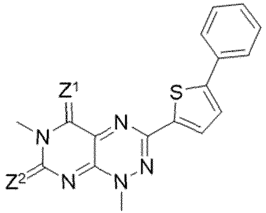 |
| 14.1 | red- phenyl-thiophenyl POG | 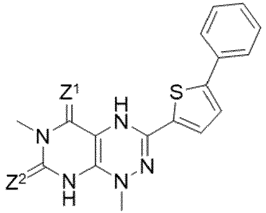 |
| 14.2 | hydroperoxy-phenyl-thiophenyl POG | 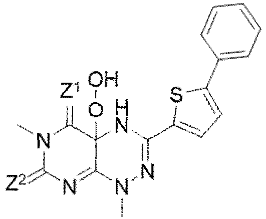 |
| 15 | N1 -iPr-styryl POG | 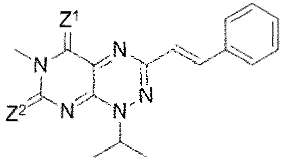 |
| 15.1 | red-N1-iPr-styryl POG | 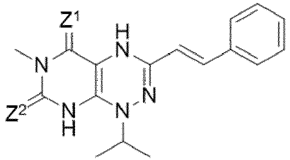 |
| 15.2 | hydroperoxy-N1-iPr-styryl POG | 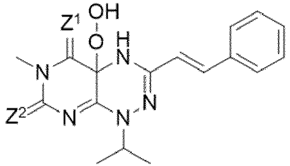 |
| 16 | N1-phenyl-styryl POG | 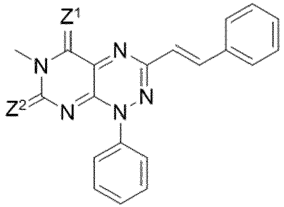 |
| 16.1 | red-N1-phenyl POG | 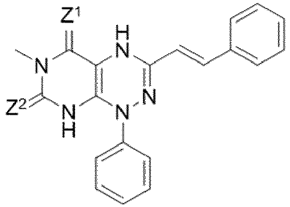 |
| 16.2 | hydroperoxy-N1-phenyl POG | 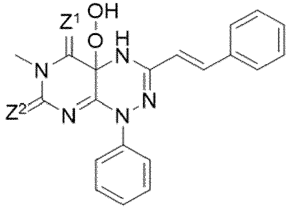 |
| 17 | N3-ethyl-styryl POG (POG-10) | 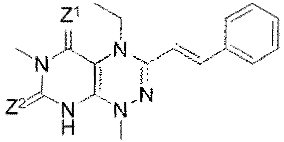 |
| 18 | thiophene POG | 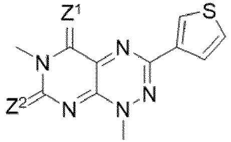 |
| 18.1 | red-thiophene POG | 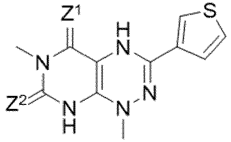 |
| 18.2 | hydroperoxy-thiophene POG | 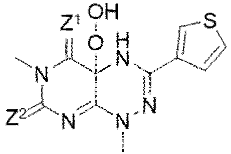 |
| 19 | di-trifluoromethylphenyl POG | 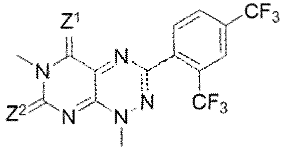 |
| 19.1 | red-di-trifluoromethylphenyl POG | 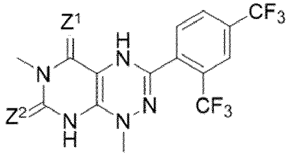 |
| 19.2 | hydroperoxy-di-trifluoromethylphenyl POG | 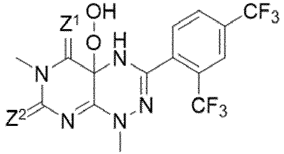 |
| 20 | styryl POG POG-4 | 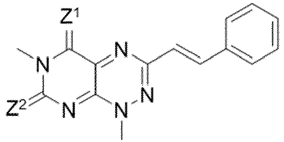 |
| 20.1 | red-styryl POG | 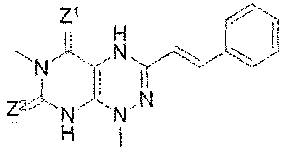 |
| 20.2 | hydroperoxy-styryl POG | 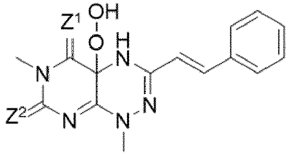 |
| 21 | N1-ethylstyryl POG | 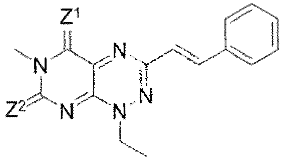 |
| 21.1 | red-N1-ethylstyryl POG | 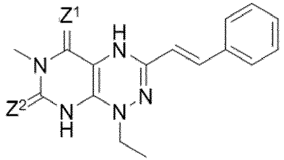 |
| 21.2 | hydroperoxy-N1-ethylstyryl POG | 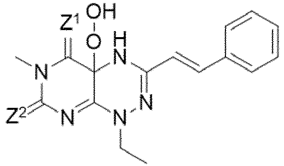 |
| 22 | hydrostyryl POG | 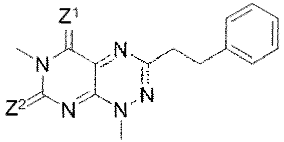 |
| 22.1 | red-hydrostyryl POG | 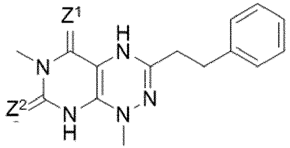 |
| 22.2 | hydroperoxy-hydrostyryl POG | 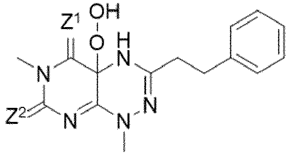 |
| 23 | benzyl POG | 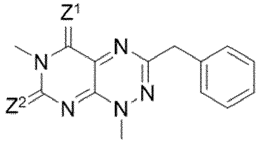 |
| 23.1 | red-benzyl POG | 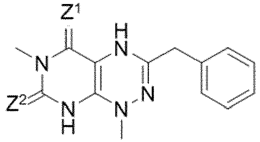 |
| 23.2 | hydroperoxy-benzyl POG | 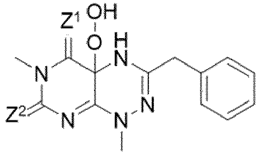 |
| 24 | N-oxide styryl POG | 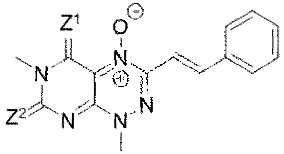 |
| 25 | phenyl POG | 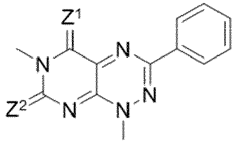 |
| 25.1 | red-phenyl POG | 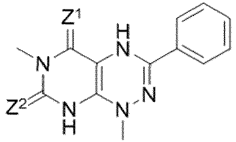 |
| 25.2 | hydroperoxy-phenyl POG | 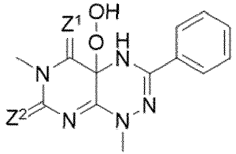 |
| 26 | naphthalene POG | 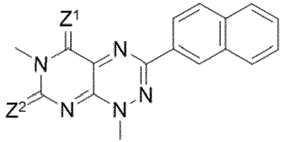 |
| 26.1 | red-naphthalene POG | 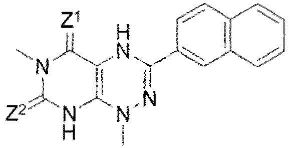 |
| 26.2 | hydroperoxy-naphthalene POG | 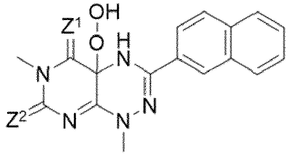 |
| 27 | p-fluorophenyl POG | 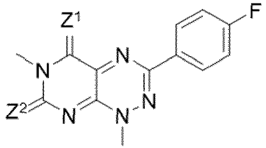 |
| 27.1 | red-p-fluorophenyl POG | 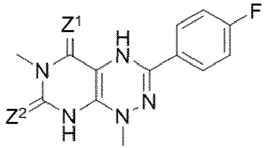 |
| 27.2 | hydroperoxy-p-fluorophenyl POG | 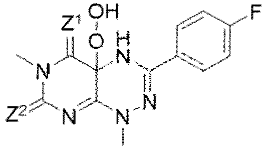 |
| 28 | 4-pyridinyl POG | 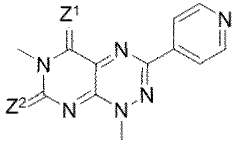 |
| 28.1 | red-4-pyridinyl POG | 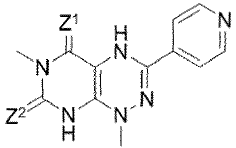 |
| 28.2 | hydroperoxy-4-pyridinyl POG | 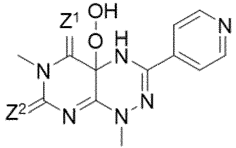 |
| 29 | 1-N-cyclopentylstyryl POG | 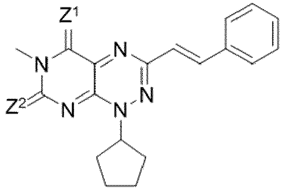 |
| 29.1 | red-1-N-Cyclopentylstyryl POG | 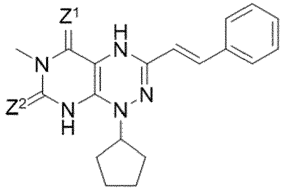 |
| 29.2 | hydroperoxy-1-N-CyclopentylstyrylPOG | 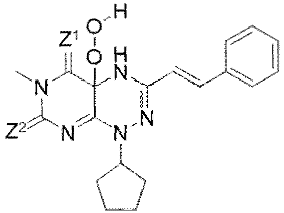 |
| 30 | 6-N-demethylstyryl POG | 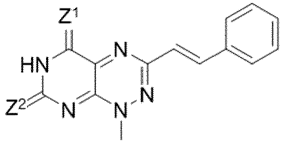 |
| 30.1 | red-6-N-demethylstyryl POG | 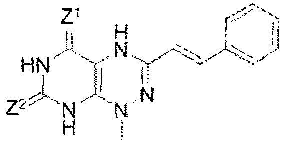 |
| 30.2 | hydroperoxy-6-N-demethylstyryl POG | 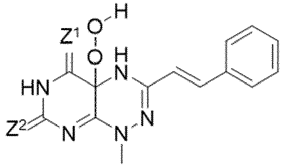 |
| 31 | 6-N-demethyl-N1-phenyl-p-nitrostyryl POG | |
| 31.1 | red-6-N-demethyl-N1-phenyl-p-nitrostyryl POG | |
| 31.2 | hydroperoxy-6-N-demethyl-N1-phenyl-p-nitrostyryl POG | |
| 32 | 6-N-demethyl-N1-phenyl-styryl POG | |
| 32.1 | red-6-N-demethyl-N1-phenyl-styryl POG | 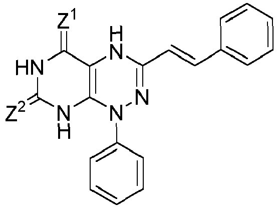 |
| 32.2 | hydroperoxy-6-N-demethyl-N1-phenyl-styryl POG | 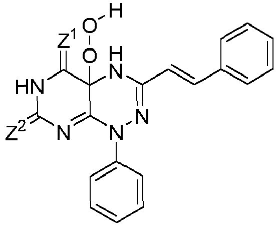 |
| 33 | N1-fluorophenyl-styryl POG | 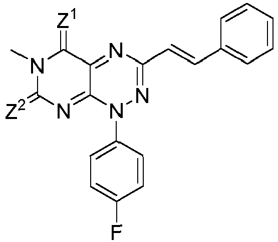 |
| 33.1 | red-N1-fluorophenyl-styryl POG | 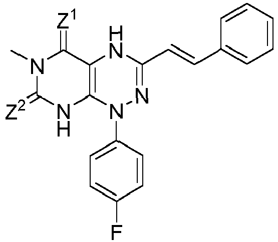 |
| 33.2 | hydroperoxy-N1-fluorophenyl-styryl POG | 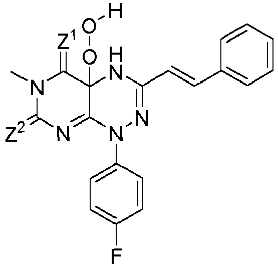 |
| 34 | N1-cyanophenyl-styryl POG | 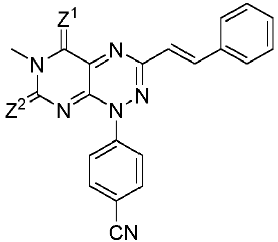 |
| 34.1 | red-N1-cyanophenyl-styryl POG | 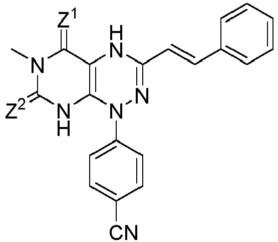 |
| 34.2 | hydroperoxy-N1-cyanophenyl-styryl POG | 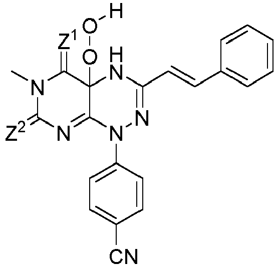 |
| 35 | p-cyanostyryl POG | 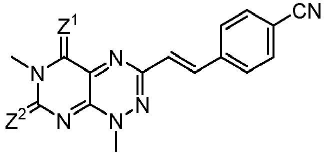 |
| 35.1 | red-p-cyanostyryl POG | 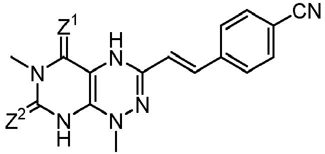 |
| 35.2 | hydroperoxy-p-cyanostyryl POG | 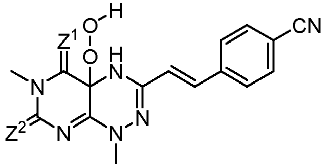 |
| 36 | 6-N-demethyl-p-nitrostyryl POG | 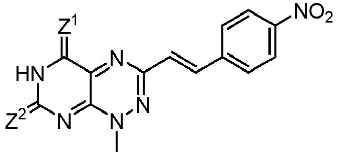 |
| 36.1 | red-6-N-demethyl-p-nitrostyryl POG | 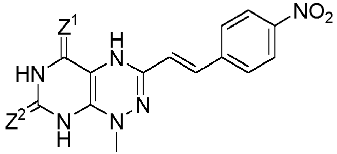 |
| 36.2 | hydroperoxy-6-N-demethyl-p-nitrostyryl POG | 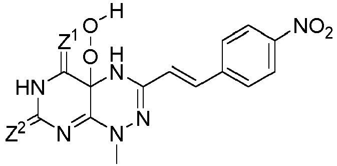 |
| 37 | N1-phenyl-N-oxide-styryl POG | 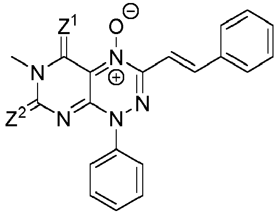 |
| 38 | 6-N-demethyl-N-oxide-styryl POG | 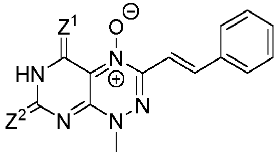 |
| 39 | p-nitro-N-oxide-styryl POG | 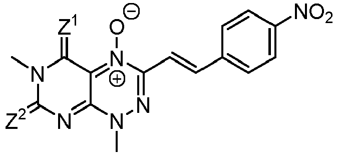 |

In the formulae of Table 1, Z¹ and Z² denote independently from each other O or S. Thus, the formulae listed in Table 1 represent four different variants (V1, V2, V3, and V4) of each example (V1: Z¹ = O and Z² = S; V2: Z¹ = S and Z² = O; V3: Z¹ = O and Z² = O; and V4: Z¹ = S and Z² = S). All variants shall be deemed to be individually disclosed in Table 1.

Generally, a senolytic can be used in the treatment of diseases or conditions in which the removal of senescent cells is beneficial. Exemplary diseases or conditions in which the removal of senescent cells is beneficial are cancer and premalignant precursor lesions, viral and/or bacterial infection diseases, neurodegenerative diseases, age-related diseases/conditions, autoimmune diseases, and inflammatory processes (such as inflammations). In an embodiment, the compound is used as senolytic medicament against any or all of these diseases/conditions.

In an embodiment, the compound is for use as senolytic against senescent cells in replicative senescence (RS) and/or in virus-induced senescence (VIS) and/or in oncogene-induced senescence (OIS),) and/or in oncogene inhibition-induced senescence (OIIS) and/or in therapy-induced senescence (TIS). In this context, it is important to make a distinction between an anti-tumor activity of POG and an anti-senescence activity of POG such as a senolytic activity against cells having oncogene-induced senescence. Notably, the so far reported anti-tumor activity of POG is only directed against proliferating tumor cells and requires high concentrations (in cell culture: > 10 µM). POG applied in such concentration would expectedly harm healthy tissue in vivo (in mice, adverse effects (in particular oxidative stress) have been observed in vivo in concentrations of > 0.5 mg POG/kg of bodyweight), i.e., such dose range is likely to be very toxic.

The senolytic activity of POG can already be exploited at much lower concentrations of less than 1 µM), hence, in vitro less than 10 % of the concentration necessary for reportedly exhibiting an anti-tumor activity in proliferating cancer cells. At low concentration (250 ug POG/kg bodyweight in mice), no relevant organ toxicity was observed in mouse models in vivo. An inhibition of never-senescent, proliferating tumor cells could not be observed at such low concentrations, while senolytic activity was evident with respect to cells being in either of the four above-mentioned states of senescence, i.e. RS, VIS, OIS, and TIS. Consequently, the compounds, the use of which is presently claimed, are particularly appropriate to be used as senolytic in case of age-related diseases, virus infections, for the prevention of tumors (in case of OIS) as well as therapeutic senolytic in the context of tumor drug therapy (in case of TIS).

In an embodiment, the compound is used in a concentration in which it does not show any activity against proliferating tumor cells. This concentration is dependent on the concrete experimental setup or the physiologic conditions of the patient to be treated, respectively. For instance, concentrations as low as 0.8 µM) produced 50 % death of senescent cells, while > 95 % of the proliferating cells remained viable under this condition. In in-vivo experiments conducted in mice, a dose of two times 250 µg POG per kg bodyweight showed a senolytic effect. Thus, in an embodiment, the concentration is 100 µg to 1000 µg POG per kg bodyweight, in particular 200 µg to 900 µg per kg bodyweight, in particular 300 µg to 800 µg per kg bodyweight, in particular 400 µg to 700 µg per kg bodyweight, in particular 500 µg to 600 µg per kg bodyweight. Typically, the concentration of the compound is at most 10 % of the reported concentration for the POG used as anti-tumor agent against proliferating tumor cells, and can be closely linked to the senescent status of the cells as indicated by the senescence-associated beta-galactosidase activity marker (SA-β-gal), i.e. it kills in a SA-β-gal-proportional manner, thereby leaving never-senescent SA-β-gal-negative cells largely unaffected. Notably, it is intrinsic to the mode of POG activation (see below) that POG is preferentially activated in the redox context of senescent cells as compared to proliferating non-senescent cells, thereby further explaining the cytotoxic efficacy of POG at a much lower concentration against senescent cells.

In an embodiment, the compound is for sequential use in treating cancer following a chemotherapeutic agent. Thus, the compound is used as senolytic for treating tumor cells in TIS. The number of tumor cells that undergo TIS during clinically applicable anti-cancer therapy (such as chemotherapy, radiation or targeted therapeutics) can be significantly higher than the number of senescent cells naturally seen to occur under other triggers. Consequently, the use of the compounds as senolytic against therapy-induced senescent tumor cells has a significantly higher likelihood to quantitatively eliminate residual cancer cells, i.e. tumor cells that survived chemotherapy or other anti-cancer therapies before (and, hence, bear the risk to account for a subsequent tumor relapse).

In an embodiment, the compound is for use in treating cancer that has previously been treated with a chemotherapeutic agent. Thus, the compound is used as senolytic for treating therapy-induced senescence of tumor cells. It could be shown that the number of tumor cells that undergo therapy-induced senescence is significantly higher than the number of senescent tumor cells that reached the status of senescence due to other triggers. Consequently, the use of the compounds as senolytic against therapy-induced senescent tumor cells has a significantly higher effect with respect to cancer therapy than the use of the compounds as senolytic against tumor cells that have reached the status of senescence due to non-therapeutic triggers.

Senescence in primary, non-malignant ("healthy") cells can be enforced in response to the expression of certain activated oncogenes. This type of senescence is referred to as oncogene-induced senescence. In a general embodiment, the compounds, the use of which is presently claimed, are used as senolytic against any senescent cells (irrespective of the trigger of senescence). In a more specific embodiment, the compounds, the use of which is presently claimed, are specifically not used as senolytic against oncogene-induced senescent cells (i.e., senescent cells that have reached their senescent status due to oncogene-induced senescence).

In an embodiment, the compounds, the use of which is presently claimed, are used as senolytic against (mutant) Ras-driven or (mutant) Braf-driven cancers, e.g., in the treatment and/or prevention of Ras-driven or Braf-driven cancers. Mutation-activated Ras genes and Braf genes are oncogenes, because they cooperate with other genetic lesions to transform normal cells into tumor cells, and to eventually develop full-blown cancer. The inventors were able to show that POG, the use of which is presently claimed, are particularly potent in killing cells that reached a status of OIS in response to Ras or Braf activation.

In an embodiment, the compounds are used for treating replicative senescence, i.e., as senolytic against cells that reached their senescent state due to replicative senescence (RS). RS cells are known as a major driver of organismic aging and age-related pathologies. Despite their relative sparsity in tissues, they account via their SASP for chronic "inflammaging", leading to age-related organ damage such as chronic obstructive pulmonary disease or liver fibrosis. Hence, eliminating pro-inflammatory RS cells by senolytics can help to slow the aging process in a system-wide fashion.

In an embodiment, the compounds are used for treating virus-induced senescence, i.e., as senolytic against cells that reached their senescent state due to virus-induced senescence. In this setting, it is - similar to RS - the therapeutic goal, to selectively eliminate senescent cells because of their contribution to inflammation-mediated organ damage, as known from the cytokine storm attributed to the SARS-CoV-2 virus in severe COVID-19, in which senolytics were shown to mitigate the course of disease.

A pharmaceutically acceptable salt of the compounds explained above is, e.g., an acid salt of a substance containing an amine or other basic group. A salt can be obtained by reacting the substance with a suitable organic or inorganic acid, such as hydrogen chloride, hydrogen bromide, acetic acid, perchloric acid and the like. Non-limiting examples of such salts are hydrochlorides, hydrobromides, sulfates, methanesulfonates, nitrates, maleates, acetates, citrates, fumarates, tartrates, succinates, benzoates and salts with amino acids such as glutamic acid. Salts of compounds containing a carboxylic acid or other acidic functional group can be prepared by reacting with a suitable base. Non-limiting examples of such salts are alkali metal salts (e.g., sodium or potassium), alkaline earth metal salts (e.g., calcium or magnesium), aluminum salts, ammonium salts, and salts of organic bases such as trimethylamine, triethylamine, morpholine, pyridine, piperidine, picoline, dicyclohexylamine, N,N'-dibenzylethylenediamine, 2-hydroxyethylamine, bis-(2-hydroxyethyl)amine, tri-(2-hydroxyethyl)amine, procaine, dibenzylpiperidine, dehydroabietylamine, glucamine, N-methylglucamine, collidine, quinine, quinoline, and basic amino acids such as lysine and arginine.

In an aspect, the present invention relates to a method of treatment, namely, a method of inducing senolysis in a patient in need thereof, wherein the method comprises administering an effective amount of a compound according to any of general formulae (I), (II), (III), or (IV) as indicated above, or a pharmaceutically acceptable salt or hydrate of such compound or derivative to the patient. In this context, the variable residues of general formulae (I), (II), (III), or (IV) have the meanings as indicated above.

In an embodiment, the method of inducing senolysis is a method of treating cancer in the patient, in particular by senolysis of senescent tumor cells that have reached their senescent state due to therapy-induced senescence, e.g., by subjecting the patient to a chemotherapy.

In an aspect, the present invention relates to an in-vitro method for senolysis. This method comprising subjecting senescent cells to a compound according to general formula (I) as indicated above, to a derivative of such compound according to any of general formulae (II), (III), or (IV) as indicated above, or to a pharmaceutically acceptable salt or hydrate of such compound or derivative. In this context, the variable residues of general formulae (I), (II), (III), or (IV) have the meanings as indicated above.

The term "in vitro" as used throughout herein also encompasses the term "ex vivo". Thus, the above explained in-vitro method for senolysis is also appropriate in ex-vivo methods. An example of such an ex-vivo method is the field of organ transplantation. Here, the senolytic compounds can be used, e.g., for preparing and optimizing organs to be transplanted (by removing senescent cells from such organs). Cell therapies such as immune therapies and cell transplantations are further examples for an ex vivo application in which senolysis can be beneficial and in which the presently described compounds can well be used as senolytic.

In an aspect, the present invention relates to a compound according to general formula (I) as indicated above, a derivative of such compound according to any of general formulae (II), (III), or (IV) as indicated above, or a pharmaceutically acceptable salt or hydrate of such compound or derivative. In this context, the variable residues of general formulae (I), (II), (III), or (IV) generally have the meanings as indicated above. However, certain limitations apply to the possible meanings of residue R² that will be explained in the following. In this context, the dashed line indicates a bond by which the residue R² is covalently bound to the triazine ring of the compound according to general formulae (I), (II), (III), or (IV).
i) R² is not 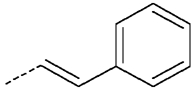 if residue R¹ is a methyl residue and residue R³ is a methyl or ethyl residue,
ii) R² is not 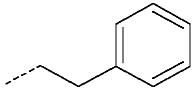 if residue R¹ is a methyl residue and residue R³ is a methyl residue,
iii) R² is not 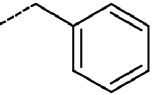 if residue R¹ is a methyl residue and residue R³ is a methyl residue,
iv) R² is not 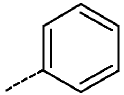 if residue R¹ is a methyl residue and residue R³ is a methyl residue,
v) R² is not 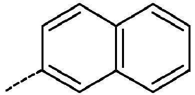 if residue R¹ is a methyl residue and residue R³ is a methyl residue,
vi) R² is not 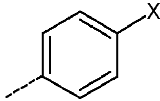 if residue R¹ is a methyl residue and residue R³ is a methyl residue, wherein X denotes F, Cl, Br, or I, and
vii) R² is not 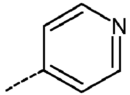 if residue R¹ is a methyl residue and residue R³ is a methyl residue.

In an embodiment, residue R² is a residue according to any of the following formulae, wherein the dashed line indicates a bond by which the residue R² is covalently bound to the triazine ring of the compound according to general formulae (I), (II), (III), or (IV):

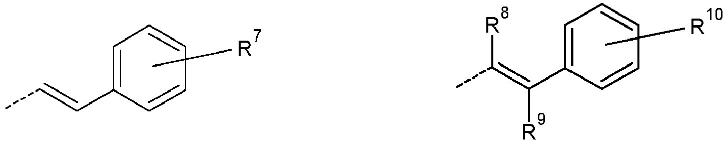

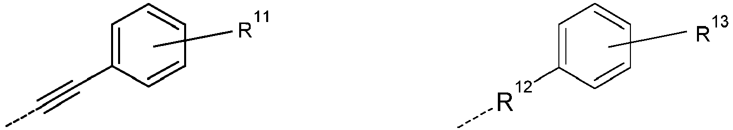

In this context, the residues R⁷, R⁸, R⁹, R¹⁰, R¹¹, R¹², and R¹³ have the meanings as indicated above. The limitations explained above with respect to possible meanings of residue R² also apply for this embodiment and the embodiments explained in the following.

In an embodiment, residue R² does not comply with any of the following formulae, wherein the dashed line indicates a bond by which the residue R² is covalently bound to the triazine ring of the compound according to general formulae (I), (II), (III), or (IV):

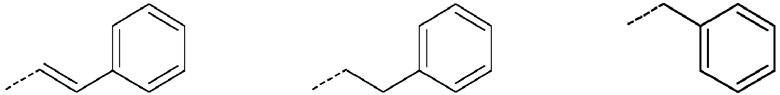

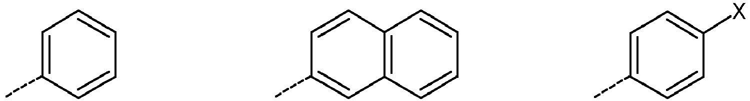

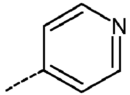

In this context, residue X has one of the following meanings: F, Cl, Br, or I, in particular F or Br, in particular F.

In an embodiment, residue R² is a residue according to any of the following formulae, wherein the dashed line indicates a bond by which the residue R² is covalently bound to the triazine ring of the compound according to general formulae (I), (II), (III), or (IV):

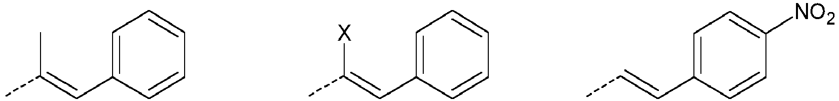

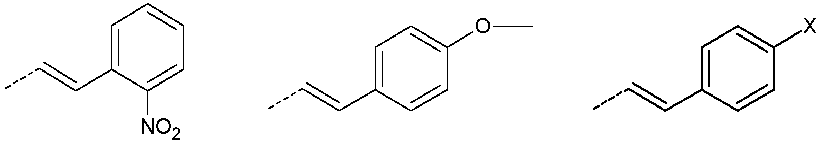

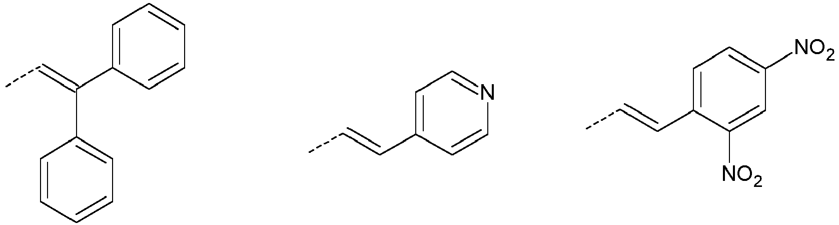

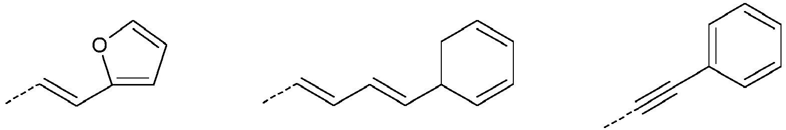

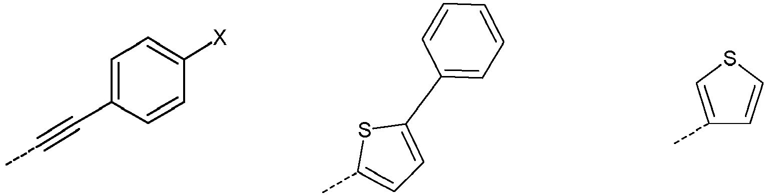

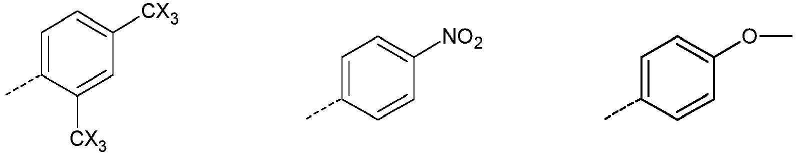

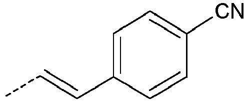

In this context, residue X has one of the following meanings: F, Cl, Br, or I, in particular F or Br, in particular F.

In an embodiment, the compound is any of compounds no. 1, 1.1, 1.2, 2, 2.1, 2.2, 3, 3.1, 3.2, 4, 4.1, 4.2, 5, 5.1, 5.2, 6, 6.1, 6.2, 7, 7.1, 7.2, 8, 8.1, 8.2, 9, 9.1, 9.2, 10, 10.1, 10.2, 11, 11.1, 11.2, 12, 12.1, 12.2, 13, 13.1, 13.2, 14, 14.1, 14.2, 15, 15.1, 15.2, 16, 16.1, 16.2, 17, 18, 18.1, 18.2, 19, 19.1, 19.2, 29, 29.1, 29.2, 30, 30.1, 30.2, 31, 31.1, 31.2, 32, 32.1, 32.2, 33, 33.1, 33.2, 34, 34.1, 34.2, 35, 35.1, 35.2, 36, 36.1, 36.2, 37, 38, and 39 (any compound being present as variant V1, V2, V3, or V4) of Table 1 listed above.

In an aspect, the present invention relates to a medicament comprising at least one compound according to the preceding explanations. The compound is typically present in the medicament in a pharmaceutically active amount. The medicament optionally comprises at least one other pharmaceutically active compound. The medicament typically also comprises pharmaceutically inactive compounds such as binders and/or excipients.

In an aspect, the present invention relates to a first method for manufacturing a compound as defined above. This method comprises the following steps:
a) adding a nitrite such as sodium nitrite to an acidic solution of 6-(1-methyl-2-((styryl)methylene)hydrazinyl)-3-methyluracil, filtering-off a precipitate, evaporating the filtrate to dryness and optionally washing it with water;
b) combining the precipitate and the dried and optionally washed filtrate and suspending the combination in an organic solvent such as ethanol;
c) adding thiophenol and stirring the mixture; filtering-off a precipitate, drying the precipitate to obtain an oxidized form of the compound, in particular ox-POG-4.

In an embodiment, the first manufacturing method further comprises the following steps:
d) adding thiophenol under inert atmosphere (such as a nitrogen atmosphere) to a suspension to the oxidized form of the compound in an organic solvent such as ethanol and stirring the mixture;
e) filtering-off the formed precipitate, optionally washing it with an organic solvent such as ethanol (in particular under an inert atmosphere such as a nitrogen atmosphere) and drying the resulting product to obtain a reduced form of the compound, in particular red-POG-4.

In an embodiment, the first manufacturing method further comprises the following steps:
f) adding 1,8-Diazabicyclo(5.4.0)undec-7-ene (DBU) to a suspension of the reduced form of the compound in an organic solvent, such as chloroform, in particular degassed chloroform;
g) adding ethyl iodide; washing the reaction mixture; drying the organic phase and obtaining another exemplary embodiment of the compound, in particular (E)-4-ethyl-1,6-dimethyl-3-styryl-4,8-dihydropyrimido[5,4-e][1,2,4]triazine-5,7(1H,6H)-dione (POG-10).

In an aspect, the present invention relates to a second method for manufacturing a compound as defined above. This method is illustrated by the following general synthesis scheme (in which the residues have the meanings as defined above) and comprises the subsequently explained steps: 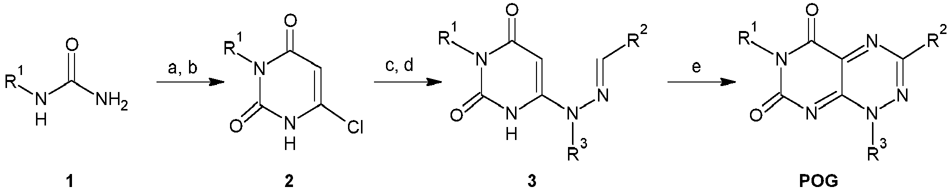
a) adding diethyl malonate and N-substituted urea **1** to a solution of sodium metal in an organic solvent such as ethanol; at least partially dissolving the formed precipitate in an organic solvent such as ethanol; dissolving the resulting solid in water or an aqueous solution and acidifying the solution; removing the solvent and filtering-off the precipitate; optionally washing the precipitate to obtain N-substituted barbituric acid;
b) adding POCl₃ to the N-substituted barbituric acid and removing excess POCl₃; pouring the product over ice water and stirring it; filtering the formed precipitate to obtain compound **2** (3-N-substituted 6-chloro-uracil 2);
c) adding substituted hydrazine to the 3-N-substituted 6-chloro-uracil **2** in an organic solvent such as ethanol; filtering-off the formed precipitate and optionally washing it to obtain 3-N-substituted 6-hydrazino-uracil;
d) adding an aldehyde to a solution of the 3-N-substituted 6-hydrazino-uracil in an organic solvent such as ethanol, optionally containing a catalytic amount of an acid such as HCl; stirring the resulting mixture; filtering-off the formed precipitate to obtain an aldehyde hydrazone **3**;
e) adding a saturated aqueous solution of NaNO₂ to a solution of the aldehyde hydrazone **3** in aqueous acidic solution such as acetic acid; stirring the reaction mixture to obtain a mixture of the compound (POG) and its 4-N oxide; isolating the 4-N oxide or adding thiophenol to the mixture, stirring the resulting mixture, and removing the solvent to obtain the corresponding compound (POG).

In an aspect, the present invention relates to a third method for manufacturing a compound as defined above, namely 4-N and 4a-substituted POG. This method is illustrated by the following general synthesis scheme (in which the residues have the meanings as defined above) and comprises the subsequently explained steps: 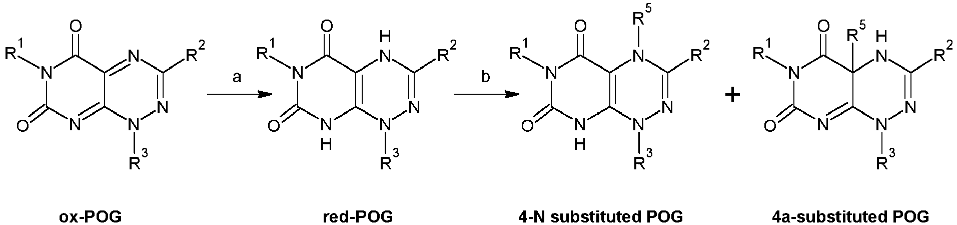
a) adding thiophenol to a suspension of an oxidized form of the compound (ox-POG) in an organic solvent, e.g., ethanol, under an inert atmosphere such as a nitrogen atmosphere; stirring the mixture; filtering-off the obtained precipitate and optionally washing it to obtain the reduced form of the compound (red-POG);
b) adding DBU to a suspension of the reduced form of the compound (red-POG) in an organic solvent such as chloroform, in particular degassed chloroform; adding an alkyl iodide and stirring the mixture; drying the product to obtain a mixture of an 4-N substituted compound and 4a-substituted compound (4-N substituted POG and 4a-substituted POG).

In an aspect, the present invention relates to a fourth method for manufacturing a compound as defined above, namely 1-N substituted POG. This method is illustrated by the following general synthesis scheme (in which the residues have the meanings as defined above) and comprises the subsequently explained steps: 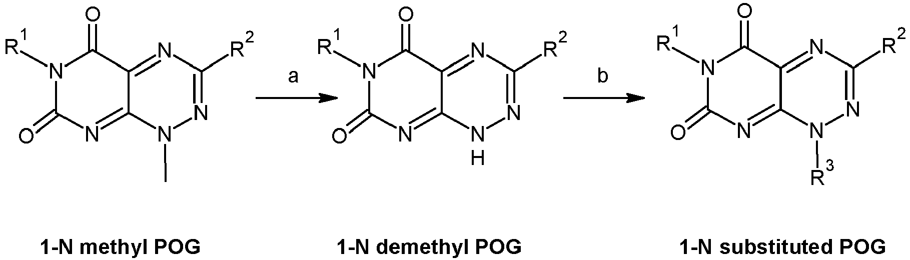
a) heating a solution of POG in an organic solvent such as dimethylformamide (DMF) to obtain 1-N demethyl POG.
b) adding anhydrous potassium carbonate or DBU to the 1-N demethyl POG in an organic solvent such as 1,4-dioxane; adding an appropriate alkyl bromide; stirring the mixture; filtering-off the precipitated potassium carbonate; extracting the mixture with an organic solvent such as ethyl acetate and drying the combined extracts to obtain the corresponding 1-N substituted POG.

In an aspect, the present invention relates to a fifth method for manufacturing a compound as defined above, namely 1-N substituted POG. This method is illustrated by the following general synthesis scheme (in which the residues have the meanings as defined above) and comprises the subsequently explained steps: 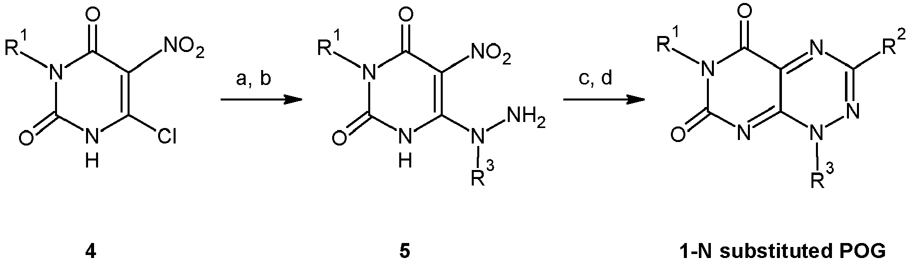
a) adding a hydrazine with a protecting group such as a tert-butyloxycarbonyl protecting group (boc) to compound **4** in an organic solvent such as DCM; stirring the mixture and filtering-off the formed precipitate; optionally washing the precipitate; drying the precipitate to obtain 3-N substituted 5-nitro 6-(2-N-Boc-hydrazino)-uracil;
b) suspending the 3-N substituted 5-nitro 6-Boc-hydrazino-uracil in an organic solvent such as DCM; adding an acid such as trifluoroacetic acid (TFA); stirring the reaction mixture and removing the solvent to obtain compound **5;**
c) adding the corresponding aldehyde to a solution of 3-N substituted 5-nitro 6-hydrazino-uracil **5** in an organic solvent such as ethanol, optionally containing a catalytic amount of an acid such as HCl; filtering-off the formed precipitate to obtain the corresponding aldehyde nitro hydrazone;
d) suspending the aldehyde nitro hydrazone in an aqueous mixture of an alcohol such as ethanol in water; adding zinc dust and a chloride such as NH₄Cl; adding an acid such as HCl; stirring the reaction mixture; filtering-off, optionally washing, and drying the formed precipitate to obtain the corresponding 1-N substituted POG derivative.

All embodiments of the uses can be combined in any desired way and can be transferred either individually or in any arbitrary manner to the compound, to the various methods, and to the medicament. Likewise, all embodiments of the compound can be combined in any desired way and can be transferred either individually or in any arbitrary manner to the use, to the various methods, and to the medicament. Furthermore, all embodiments of the methods can be combined in any desired way and can be transferred either individually or in any arbitrary manner to the compound, to the use, and to the medicament. Finally, all embodiments of the medicament can be combined in any desired way and can be transferred either individually or in any arbitrary manner to the use, the compound, and to the various methods.

Further details of aspects of the present invention will be explained in the following making reference to exemplary embodiments and accompanying Figures. In the Figures:
- Figure 1A: shows a scheme on the mechanism of action of POG in senescent cells;
- Figure 1B: is an overview illustrating the pan-senolytic activity of POG in all types of cellular senescence;
- Figure 2A: shows a plot illustrating the generation of hydrogen peroxide by POG in a fluorescence-based europium tetracycline assay at different POG concentrations;
- Figure 2B: shows a plot illustrating the generation of hydrogen peroxide by POG in a fluorescence-based europium tetracycline assay at different NADH concentrations;
- Figure 3: shows a plot illustrating the redox potential of cells driven into senescence;
- Figure 4: shows two plots illustrating the viability of non-senescent and senescent cells after treatment with POG;
- Figure 5: shows a plot illustrating the effect of POG in non-senescent cells with differing redox potential;
- Figure 6: shows a plot illustrating the senolytic activity of the known senolytic Navitoclax;
- Figure 7A: shows a plot illustrating the effect of POG on physical performance in aged mice;
- Figure 7B: shows a plot illustrating the outcome of a POG-assisted anticancer treatment *in vivo;*
- Figure 8A: shows a first plot illustrating the effect of POG on the number of senescent liver cells of mice of different ages;
- Figure 8B: shows a second plot illustrating the effect of POG on overall viability of liver cells isolated from mice, wherein POG selectively kills only the senescent subpopulation isolated from aged mice;
- Figure 9A: shows a first plot illustrating the effect of POG on N-nitroso-N-methylurea-(NMU)-induced senescent lesions in mouse embryo fibroblasts;
- Figure 9B: shows a second plot illustrating the effect of POG on NMU-induced cell transformation of mouse embryo fibroblasts;
- Figure 10: shows a plot illustrating the effect of POG on DMBA-induced OIS cells (mouse embryo fibroblasts);
- Figure 11: illustrates the effect of POG on NMU-induced tumorigenesis in vivo;
- Figure 12: illustrates the senolytic activity of POG against virus-induced senescence (VIS); and
- Figure 13: illustrates the generation of hydrogen peroxide by POG and phenylethine POG in the presence of DTT.

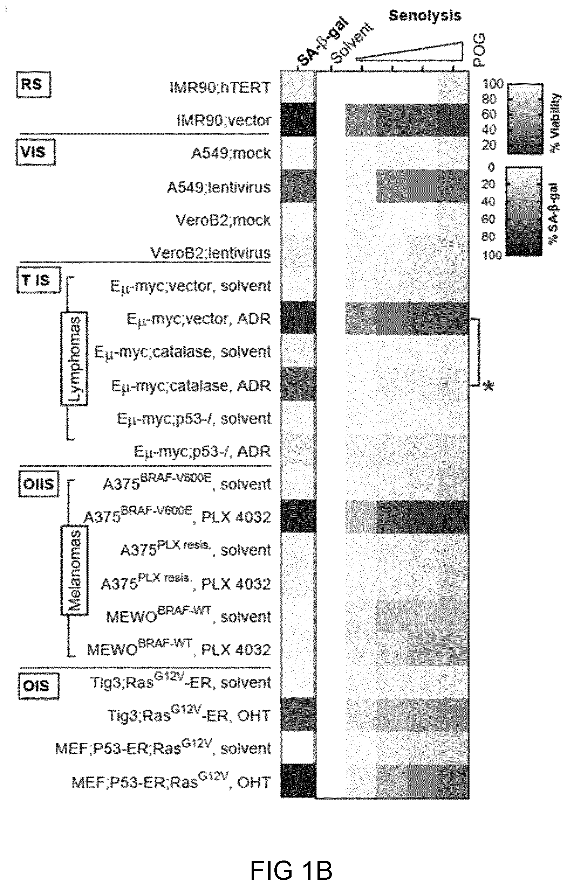

Figure 1 shows a scheme on the mechanism of action of POG in senescent cells. As already explained above, the oxidized form of POG (ox-POG) can be reduced to the reduced form of POG (red-POG) in the presence of a suitable biochemical reducing agent such as DTT or NADH. Red-POG then forms a hydroperoxy species with molecular oxygen. The original form of POG is recovered by release of hydrogen peroxide. The released hydrogen peroxide leads to apoptosis of the senescent cells.

The redox potential of senescent cells (i.e., the concentration of NADH or the NADH/NAD⁺ ratio) is significantly higher than the redox potential of non-senescent cells, thereby preferentially activating POG in senescent as compared to proliferating never-senescent cells and explaining, at least in part, the preferential cytotoxic action of POG in senescent cells.

Non-tumor cells enter a state of senescence by natural ageing (so-called replicative senescence (RS)) or due to a (high-titer) virus infection (so-called virus-induced senescence (VIS)). Primary, non-malignant cells can develop senescence when an activated oncogene such as mutant Ras or BRaf is expressed in these cells (so-called oncogene-induced senescence (OIS)).

Tumor cells can be transferred to a senescent state by chemotherapy or radiation therapy (so-called therapy-induced senescence (TIS)).

In a senescent state, the cells show increases in glycolysis, the tricarboxylic acid cycle (TCA cycle) metabolism, and lipid oxidation. These factors lead to increased cellular NADH levels and thus to an altered redox potential of the cells. The oxidative stress elicited in senescent cells results in the depletion of the cellular GSH pool. Both the elevated NADH- and massively decreased GSH levels present in senescent cells result in net activation of POG (that is production of cytotoxic H₂O₂) selectively in senescent cells.

The pan-senolytic effect of POG in all types of cellular senescence identified by the inventors is illustrated in Figure 1B that will be explained in the following paragraphs.

*RS*: Low-passage human lung fibroblasts (IMR-90) were stably transduced with ana RS-ablating hTERT-overexpressing or an empty vector as a control, and subsequently passaged until the vector-transduced cells had quantitatively entered RS at passage number 53. Increased passage numbers reflect the need of replating (repassaging) the cells due to their proliferative capacity, which was exhausted here at passage 53.

*VIS*: The human alveolar epithelial adenocarcinom cell line A549 as well as VIS-deficient VeroB4 cells were infected with a high-titer GFP-expressing lentivirus (pCDH-CMV-MCS-EF1-GFP; MOI = 50) to induce VIS.

*TIS*: Eµ-myc;Bcl2 lymphoma cells (with or without catalase overexpression) and senescence-/p53-deficient Eµ-myc; Bcl2 lymphoma cells were treated with ADR (0.02 µg/ml) or solvent.

*OIIS:* A375^{BRafV600E}, A375^{BRafV600E;PLX4032-resistant} and MEWO^{BRaf-WT} melanoma cell lines were exposed to PLX4032 (1.5 µM) or solvent (WT = wild-type).

*OIS*: ER:Ras^{G12v} Tig3 cells were treated with OHT or solvent as in C. Primary mouse embryo fibroblasts (MEF) harboring ana OHT-inducible wild-type-p53 allele were treated with or without OHT and subsequently transduced with a Ras^{G12V} overexpressing pBABE vector to induce OIS only in OHT-treated wild-type-p53 cells.

All cells were analyzed at day 10 after start of the treatment and oncogene induction, respectively. Shown in grey in the first (left) column are mean percentages of senescence-associated beta-galactosidase (SA-β-gal)-)positive cells for each treatment. Proliferating cells and their senescent counterpart were exposed to the same increasing concentrations of POG (from left to right: murine Eµ-myc lymphoma cells and primary mouse fibroblasts = 0, 0.2, 0.4, 0.8, and 1.2 µM) POG, for VIS and RS = 0, 2, 3, 4, and 5 µM) POG, and for all others = 0, 1, 2, 3, and 4 µM) POG) for 22 h before measuring their viabilities using a dye exclusion test.

Viability of solvent-treated cells were set to 100% for each treatment and viabilities of POG-treated cells were normalized relative to solvent-treated controls. The asterix indicates lymphoma cells engineered to overexpress catalase that are capable to enter TIS but lack sensitivity to POG.

### Quantification of hydrogen peroxide generated by peroxygenins

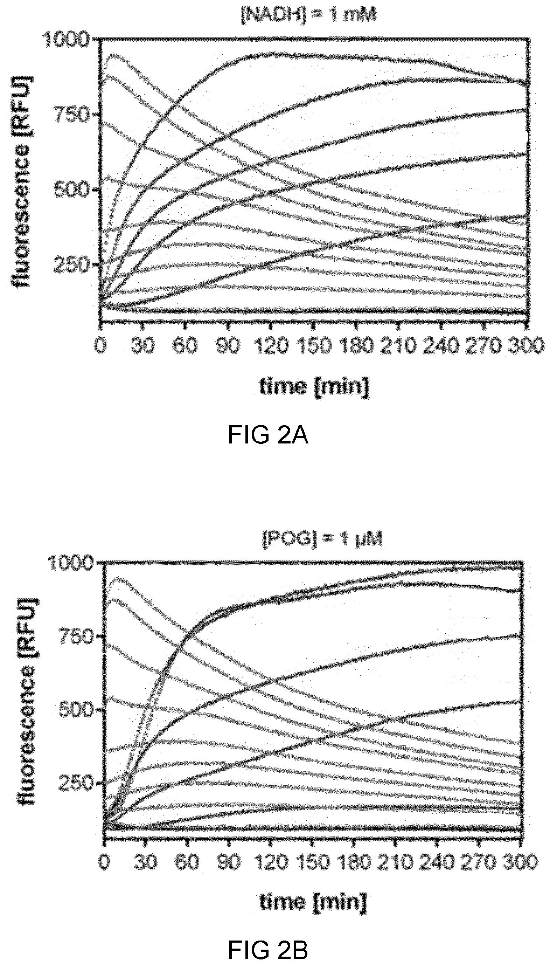

For a confirmation of the POG redox cycling, hydrogen peroxide formed by peroxygenin from elemental oxygen was quantified. For this purpose, a fluorescence-based europium tetracycline assay in 3-(N-morpholino)propanesulfonic acid (MOPS) buffer at pH 6.9, close to the physiological pH, was adapted for monitoring of hydrogen peroxide released from POG-4 (Wolfbeis et al., 2002; Dürkop et al., 2005; Wu et al., 2005; Schrenkhammer et al., 2007) Reportedly, this assay is based on the formation of an quaternary pentagonal bipyramidal Eu(III)-tetracycline (Tc) complex of the stoichiometry Eu₂Tc₂ (Karthikeyan et al., 2004) in which one equivalent of hydrogen peroxide forms a bridged peroxide complex (µ-H₂O₂ ligand) (Dehaen et al. 2009) and upon excitation at 405 nm fluoresces at 616 nm. First, the sensitivity of the assay toward hydrogen peroxide was investigated. For that, different hydrogen peroxide concentrations, ranging from 10 µM to 1000 µM, were used to analyze the fluorescence signal (Figures 2A and 2B, curves 6 to 14 and 26 to 34; curves 6 and 26:1000 µM, curves 7 and 27: 800 µM, curves 8 and 28: 400 µM, curves 9 and 29: 200 µM, curves 10 and 30: 100 µM, curves 11 and 31: 50 µM, curves 12 and 32: 25 µM, curves 13 and 33: 10 µM, curves 14 and 33: 0 µM).

Next, the same assay conditions were applied to quantify hydrogen peroxide formation from POG-4 (V3) (referred to in the following simply as POG-4) having the following formula:

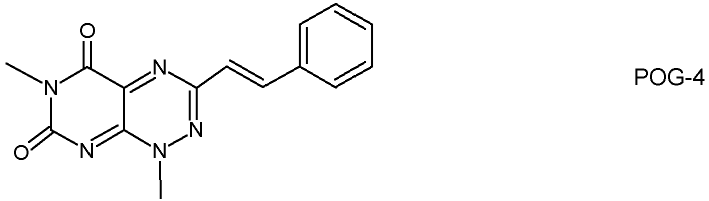

POG-4 was added to the assay buffer at various concentrations (Figure 2A; curve 1: 5 µM, curve 2: 2 µM, curve 3: 1 µM, curve 4: 0.5 µM, curve 5: 0.1 µM with 1 mM NADH. In contrast to pure hydrogen peroxide, incubation of POG-4 with NADH produced a steadily increased fluorescence, suggesting the continuous generation of H₂O₂ during the observed time frame of 300 min. Higher concentrations of POG-4 led to more rapidly growing and higher fluorescence intensities, corresponding to higher amounts of generated hydrogen peroxide.

At a concentration of 5 µM POG-4, the maximum concentration was reached at approximately 120 minutes and then slightly decreased.

Comparing the time-dependent generation of fluorescence signals from H₂O₂ (curves 6 to 14) and from POG-4 (curves 1 to 5) it can be concluded that the formation of the Eu₂Tc₂-H₂O₂ complex is significantly faster than the generation of hydrogen peroxide by POG-4.

Figure 2B shows the result of an equivalent experiment in which the POG concentration was kept constant at 1 µM, wherein the NADH concentration was varied from 0.1 mM to 5 mM (Figure 2B; curve 21: 5 mM, curve 22: 2 mM, curve 23: 1 mM, curve 4: 0.5 mM, curve 5: 0.1 mM). A higher NADH concentration generally resulted in a higher hydrogen peroxide concentration, wherein the hydrogen peroxide formation curves of 2 mM NADH and 5 mM NADH were quite similar (within the limits of measurement accuracy).

The senolytic effects of POG-4 were investigated in several established senescence models. In these models, cells were driven into therapy-induced senescence (TIS), virus-induced senescence (VIS), or replicative senescence (RS).

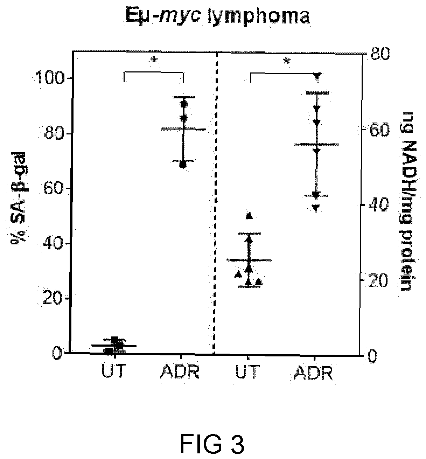

In a TIS-model, it could be shown that senescent cells indeed contained increased NADH levels compared to the control (Figure 3 illustrating an Eµ-myc cell line after TIS with doxorubicin (Adriamycin, ADR) and control (UT).

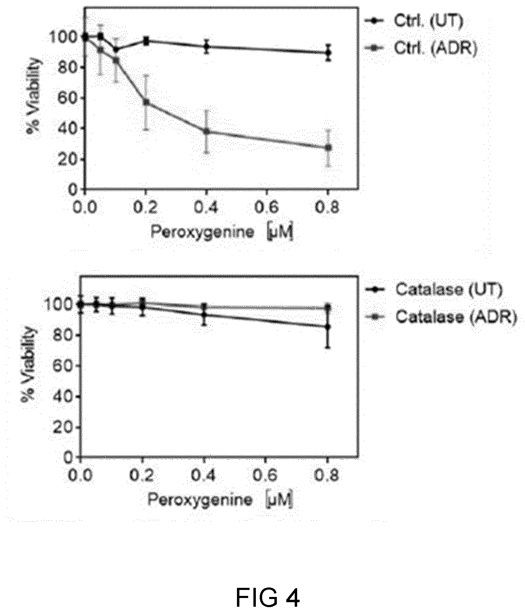

The senescent state was documented by specific measurement of increased SA-β-galactosidase activity. In the same TIS model, senescent cells were found to be significantly more sensitive to POG-4 than non-senescent tumor cells, highlighting the selectivity of the presently claimed and described senolytics for senescent cells. Figure 4 shows the results of a 22-hours cytotoxicity assay visualizing the difference in viability rate between senescent cells treated with POG-4 (ADR) and non-senescent cells treated with POG-4 (UT) (upper panel). Eµ-myc;Bcl2 lymphoma cells (with or without ectopic catalase overexpression, introduced via stable retroviral gene transfer) and senescence-/p53-deficient Eµ-myc;Bcl2 lymphoma cells were treated with ADR (0.02 µg/ml) or solvent for 10 d. Proliferating cells and their senescent counterpart were exposed to the same increasing concentrations of POG for 22 h before measuring their viabilities using a dye exclusion test (Ghost Dye Red 780, 13-0865-T500, Tonbo). Viability of solvent-treated cells were set to 100% for each treatment and viabilities of POG-treated cells were normalized relative to solvent-treated controls.

The effect was shown to be based on the action of hydrogen peroxide, as the POG-4 did not exhibit senolytic activity when the senescent cells were protected against hydrogen peroxide by the expression of catalase, an enzyme cleaving hydrogen peroxide (lower panel of Figure 4).

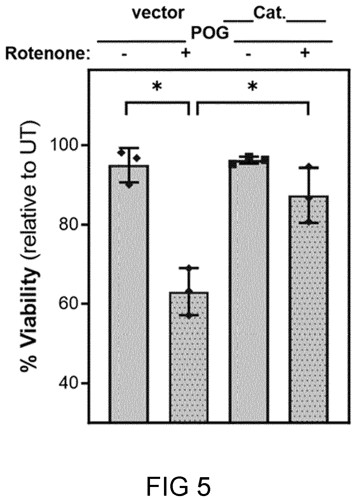

Furthermore, it was demonstrated that the activity of POG-4 relied on increased levels of the reducing equivalent (NADH) in cells. Increasing cellular NADH levels sensitizes cells towards POG-mediated killing in a H₂O₂-dependent fashion. Primary mouse embryo fibroblasts (MEF) were stably transduced with a catalase-overexpressing retrovirus or empty virus and exposed to 500 nM rotenone (an NADH dehydrogenase inhibitor used to increase cellular NADH levels via inhibition of the mitochondrial complex I) 2 h prior to POG exposure (1 µM) before measuring cell viabilities 22 h later using a dye exclusion assay (Ghost Dye Red 780, 13-0865-T500, Tonbo). Figure 5 illustrates the results in form of means ± standard deviation (SD).

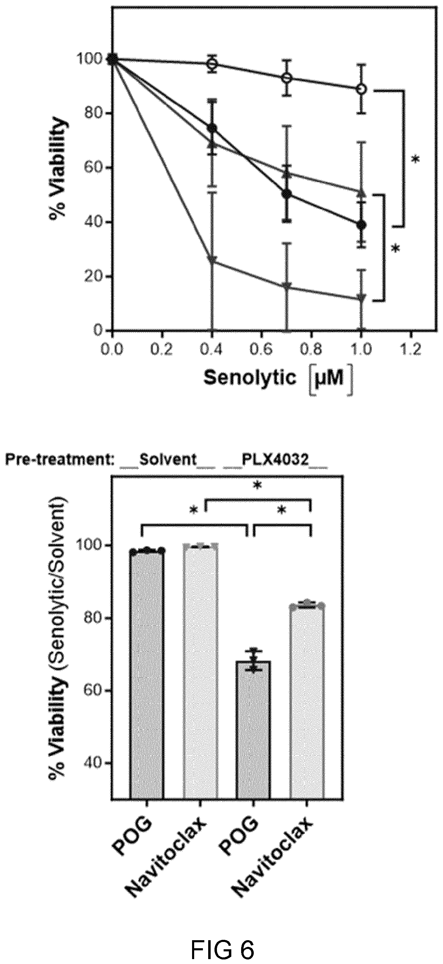

For a direct side-by-side comparison of the senolytic activities of POG-4 and Navitoclax in TIS and OIIS (oncogene-inhibition induced senescence), respectively, murine Bcl2-protected Eµ-myc lymphoma cells (n = 4, upper panel of Figure 6) as well as the human BRafV600E mutant melanoma cell line A375 (lower panel of Figure 6) were treated for 10 d with either ADR (0.02 µg/ml) or PLX4032 (1.5 µM) (an inhibitor of the mutant B-Raf enzyme) or its solvents. Induction of senescence was verified using the SA-β-gal assay before exposing cells with POG. Cell viabilities were measured after 22 h using the dye exclusion assay (Ghost Dye Red 780, 13-0865-T500, Tonbo). Figure 6 illustrates the results in form of means ± standard deviation (SD). In the upper panel of Figure 6, unfilled circles denote proliferating cells treated with POG-4, filled circles denote senescent cells treated with POG-4, triangles standing on the base denote proliferating cells treated with Navitoclax, and standing on the apex denote senescent cells treated with Navitoclax.

To quantify the global impact of POG on age-related physical decline, a rotarod performance test was conducted, which captures the time mice can run on a rotating rod before falling off due to exhaustion. As expected, older DMSO solvent-only-exposed control mice (10-months old) dropped after much shorter periods off the rod compared to untreated young mice (10-weeks old), while POG administration significantly extended the running time achieved in the older age group, albeit not to the extent seen in young miceFigure 7A; solvent: solvent-treated mice; POG: POG-4-treated mice).

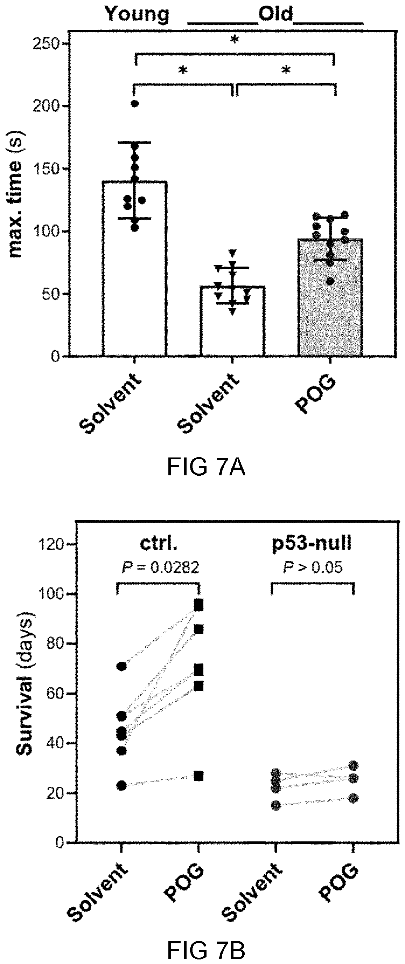

For the experiments, the results of which are shown in Figure 7A, C57/BL6 mice (10-months old, "old mice") were treated twice with POG (0.25 mg/kg body weight, *i.v.* injected every fortnight; *n* = 11) or solvent (*n* = 11), before their physical performance was assessed using a rotarod device one month later (from 4 to 40 rpm in 180 s; acceleration rate: 0.2 rpm/s). For comparison a cohort of untreated young mice (8-weeks old; *n* = 10) was measured in parallel. Each datapoint represents the maximum time the (previously trained) animal was able to remain on the rotating rod from 5 consecutive runs per mouse.

The inventors also analyzed the impact of a sequential POG exposure on overall survival in chemotherapy-exposed mice bearing senescence-capable *vs*. senescence-incapable lymphomas. Strikingly, most mice harboring Eµ-*myc* transgenic lymphomas generated from a *p53*^{+/+} background as a senescence-capable control lived significantly longer, when POG, (2 × 0.25 mg/kg bodyweight), as compared to DMSO only, was administered following exposure to the senescence-inducing chemotherapeutic agent cyclophosphamide (CTX). Mice bearing senescence-incapable Eµ-*myc* lymphomas failed to realize such benefit due to lack of both *p53* alleles (Figure 7B). In essence, POG treatment led to reduction of senescent cells *in vivo,* and extended healthy lifespan of aged and anticancer-treated mice.

Figure 7B shows the overall survival of 7-day CTX-treated Bcl2-protected Eµ-myc (*n* = 7) or Eµ-myc;p53-null (*n* = 4) lymphoma-bearing mice randomly assigned to sequential exposure to POG or solvent, presented as matched pair analysis (*P* = 0.0282 for Eµ-myc comparison; *P* > 0.05 for Eµ-myc.p53-/- comparison). CTX treatment: 1 × 300 mg/kg body weight via *i.p.,* POG treatment: 2 × 0.25 mg/kg body weight via the *i.v.* application route. Figure 7B illustrates means ± SD, wherein an unpaired two-tailed Student's t test was done for statistical analysis (**P* < 0.05).

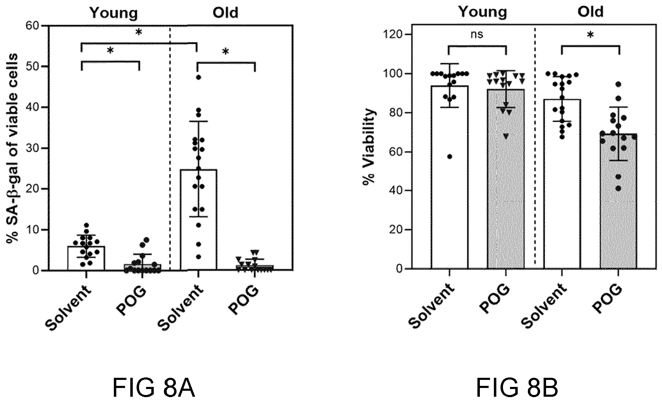

Additionally, the inventors were able to show that the percentage of senescent liver cells from old mice decreased significantly upon treatment with POG-4 (Figures 8A and 8B; Solvent: non-treated mice; POG: POG-4-treated mice) due to the senolytic effect of POG-4. Liver cell preparations from young (10-weeks old) and old (20-months old) C57/BL6 mice were treated *ex vivo* for 22 h with POG (1.5 µM) or solvent. Cells were fixed and stained for SA-β-gal, TUNEL (Terminal deoxynucleotidyl transferase dUTP nick end labeling) and DAPI (4',6-diamidino-2-phenylindole). Percentage of senescent (SA-β-gal-positive) cells present in the viable (TUNEL-negative) liver cell population (Figure 8A) and overall viability of the liver cell population (Figure 8B) was quantified via fluorescence microscopy.

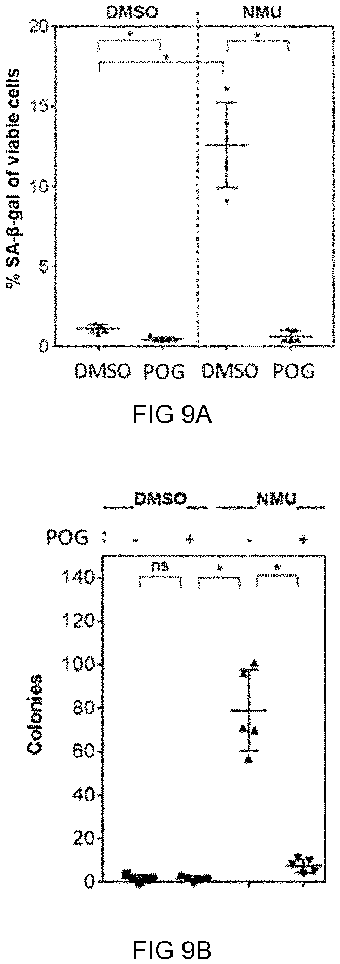

The inventors found that POG-4 is not only able to kill senescent tumor cells but that it is capable to prevent Ras-driven tumor formation in-vivo (Figures 9A, 9B, 10, and 11).

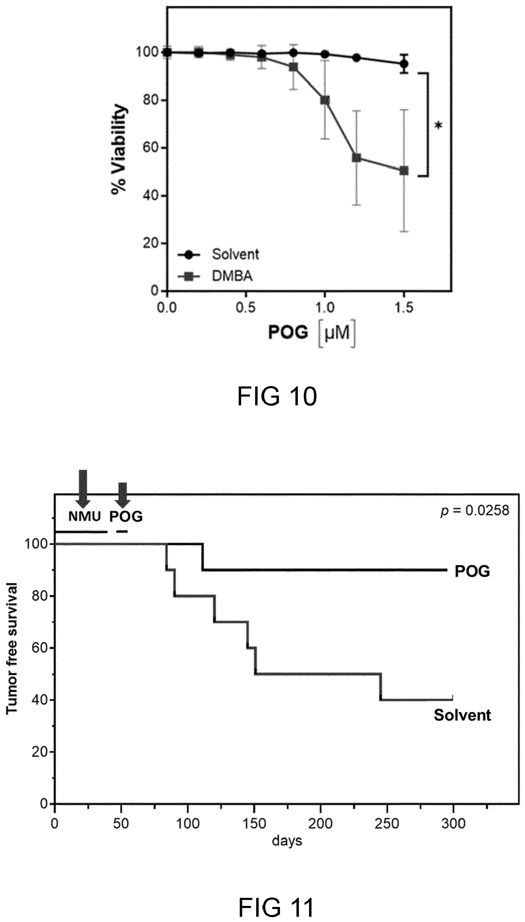

Primary mouse fibroblasts (MEF) were treated for 5 weeks with 2 mM N-nitroso-N-methylurea (NMU) or solvent to mutagenize their genomes. Selective killing of senescent subpopulation was assessed by measuring the percentage of SA-β-gal positive cells after POG-4 exposure (1.0 µM for 22 h). Figure 9A shows means ± standard deviation (SD). Figure 9B shows that POG-4 ablates NMU-induced transformation of primary MEF in vitro. 2 × 10⁴ of viable NMU-pre-treated MEF with or without subsequent POG-4 exposure (1.0 µM) from the experiment of Figure 9A were seeded in 0.3 % soft-agar and cultivated for 10 weeks, before formation of colonies (>50 cells/colony) was quantified. Again, means ± SD are illustrated
POG-mediated senolysis of DMBA-induced OIS cells was tested; the results are depicted in Figure 10. Like NMU, 7,12-dimethyl-benzoanthracen (DMBA) is known to induce oncogenic mutations across all Ras isoforms. MEF were mutagenized with DMBA (0.25 µg/ml) for 7 days before cells were exposed to increasing concentrations of POG 1 week later. Cell viabilities were measured 22 h later using a dye exclusion assay (right; (Ghost Dye Red 780, 13-0865-T500, Tonbo). SA-β-gal stainings (left) were performed 2 days after POG exposure. Filled circles denote experiments conducted with solvent; filled squares denote experiments conducted with DMBA.

The interference of POG-4 with NMU-induced tumorigenesis was established *in vivo* (Figure 11). For this purpose, 30 days old C57/BL6 mice were mutagenized for 5 consecutive weeks with 30 mg NMU/kg bodyweight (b.w.) intraperitoneally (i.p.) prior to treatment with POG-4 (2 × 0.25 mg kg⁻¹ body weight intravenously (i.v.) injected within one week) or solvent, respectively. The mice were subsequently monitored for 300 d and T-cell lymphoma development was plotted as the time between first NMU treatment and tumor manifestation in a Kaplan Meier format. Log-rank [Mantel-Cox] test was used to compare solvent- and POG-treated cohorts. Figure 11 illustrates that POG-4 interferes with NMU-induced tumorigenesis in vivo (p = 0.0258).

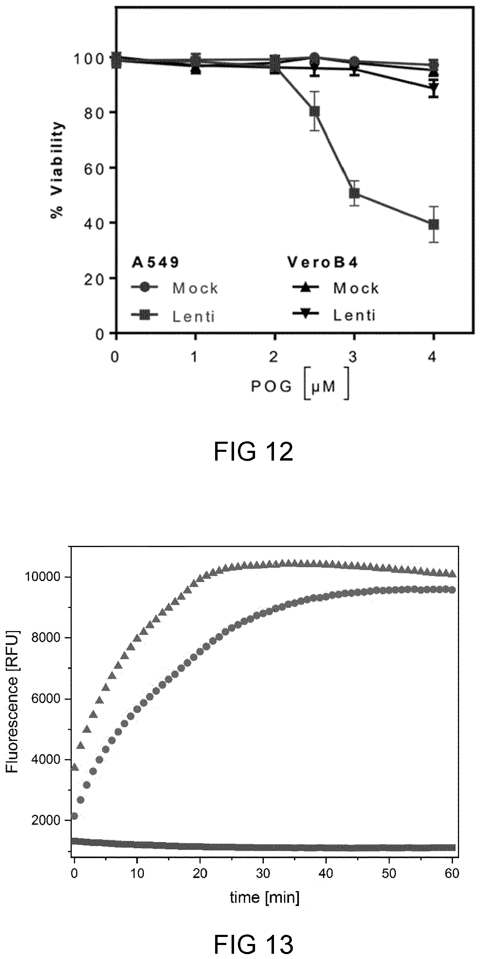

POG-4 was also found to exhibit senolytic activity against virus-induced (VIS) senescence (Figure 12). The adenocarcinomic alveolar epithelial cell line A549 as well as VIS-deficient VeroB4 cells were infected with a high-titer GFP-expressing lentivirus (pCDH-CMV-MCS-EF1-GFP; multiplicity of infection [MOI] = 50). At day 6, the cells were stained for SA-β-gal before treating them with increasing concentrations of POG-4. After 22 h, the cell viability was measured using a dye exclusion test. Figure 12 illustrates means ± SD.

The experiments explained in the preceding paragraphs prove that peroxygenins act effectively as pan-senolytics. While most of the experiments were conducted with POG-4 (also denoted as styryl POG or styrenyl POG, i.e., a POG containing a styrenyl residue in position R²), many novel, hitherto unreported POG derivatives like phenylethinyl POG (also referred to as ethine POG; confer structure no. 12 in Table 1) as well as all other POG listed in Table 1 were synthesized and many of them were successfully tested. Phenylethinyl-POG was found to be more potent in the generation of hydrogen peroxide than styrenyl POG in the EuTc assay (Figure 13). In this assay, hydrogen peroxide generation by 500 nM POG-4 (filled circles) or by 500 nM phenylethinyl POG (filled triangles) in the presence of 1 mM DTT was compared. A blank sample is illustrated by filled squares.

### Materials and methods

### Synthesis protocol 1

### (E)-1,6-Dimethyl-3-styrylpyrimido[5,4-e][1,2,4]triazine-5,7(1H,6H)-dione (ox -POG-4)

Sodium nitrite (2.3 g, 34 mmol) was added at 0 °C to a solution of 6-(1-methyl-2-((styryl)methylene)hydrazinyl)-3-methyluracil (8.0 g, 28 mmol) in glacial acetic acid and water. After 2 h the precipitate was filtered off, the filtrate was evaporated to dryness and washed with little water. Both residues were combined, suspended in EtOH and thiophenol (approx. 2 eq. thiophenol per N-oxide present in the mixture) was added. The mixture was stirred for 45 min at 90 °C. The mixture was allowed to cool to room temperature and the red precipitate was filtered off and dried in vacuo to afford ox-POG-4 (5.7 g, 19 mmol, 69%).
¹H-NMR (300 MHz, DMSO-*d₆*): δ 7.80-7.75 (m, 3H), 7.47-7.39 (m, 3H), 7.29 (d, 1H, *³J* = 16 Hz), 3.98 (s, 3H), 3.28 (s, 3H).
¹³C-NMR (75 MHz, DMSO-*d₆*): δ 158.9, 154.0, 151.9, 149.3, 146.0, 137.4, 135.0, 129.6, 128.9, 127.8, 122.0, 42.4, 28.3
HRMS (ESI) [*m*/*z*]: calculated: 296.1142 (M+H)⁺; found: 296.1141 (M+H)⁺
CHN analysis: calculated N 23.7, C 61.0, H 4.4; found: N 23.7, C 61.7, H 4.5.
Absorption maximum: 329 nm, extinction coefficient: 93109.1 L/mol*cm (329 nm).

### (E)-1,6-Dimethyl-3-styryl-4,8-dihydropyrimido[5,4-e][1,2,4]triazine-5,7(1H,6H)-dione (red-POG-4)

Thiophenol (405 µL, 3.9 mmol) was added under nitrogen atmosphere to a suspension of ox-POG-4 (499 mg, 1.7 mmol) in degassed EtOH. The mixture was stirred 3 h at room temperature. The dark brown precipitate was filtered off, washed three times with degassed EtOH under nitrogen and dried in vacuo to afford red-POG-4 (440 mg, 1.5 mmol, 88%).
¹H-NMR (300 MHz, DMSO-*d₆*): δ 10.95 (s, 1H), 7.63-7.24 (m, 6H), 6.93 (s, 1H), 6.46 (d, 1H, *³J* = 16 Hz), 3.09 (s, 3H), 2.98 (s, 3H).
Due to oxidation sensitivity, no LCMS analysis could be made.
Absorption maximum: 298 nm, extinction coefficient: 93327.3 L/mol*cm (298 nm).

### (E)-4-Ethyl-1,6-dimethyl-3-styryl-4,8-dihydropyrimido[5,4-e][1,2,4]triazine-5,7(1H,6H)-dione (POG-10)

1,8-Diazabicyclo(5.4.0)undec-7-ene (DBU) (670 µL, 4.5 mmol) was added to a suspension of red-POG-4 (670 mg, 2.3 mmol) in degassed CHCl₃. After 10 min ethyl iodide was added and stirred for 16 h at 60 °C. The reaction mixture was concentrated, and the remaining chloroform was washed three times each with 1M hydrochloric acid, brine and water. The organic phase was dried over sodium sulfate and evaporated to dryness. The residual was purified by preparative HPLC (water/MeCN) to afford POG-10 (54 mg, 0.166 mmol, 7%) as yellow powder.
¹H-NMR (300 MHz, DMSO-*d₆*): δ 10.45 (s, 1H), 7.59-7.52 (m, 3H), 7.47-7.40 (m, 3H), 6.85 (d, 1H, *³J* = 16 Hz), 4.09 (dq, 1H, *²J* =12 Hz, *³J* =7.0 Hz), 3.61-3.50 (m, 4H), 3.18 (s, 3H), 1.24 (t, 3H, *³J* = 7.0 Hz).
¹³C-NMR (75 MHz, DMSO-*d₆*): δ 157.9, 154.2, 151.2, 137.8, 136.8, 134.6, 129.8, 129.1, 127.5, 117.2, 101.4, 63.7, 57.0, 27.6, 8.7
HRMS (ESI) [*m*/*z*]: calculated: 326.1612 (M+H)⁺; found: 326.1625 (M+H)⁺

### Synthesis protocol 2

### General synthesis scheme

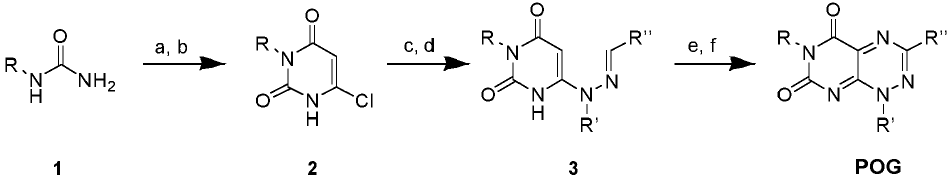

Reaction conditions: (a) Na, EtOH, diethyl malonate, N-substituted urea 1, µW, 170 °C, 6 min; (b) POCl₃, µW, 140 °C, 5 min; (c) R'NHNH₂, EtOH, 100 °C, 10 min; (d) R"CHO, EtOH, HCl, room temperature (rt), 1 h (e) NaNO₂, AcOH, H₂O, 0 °C to rt, over night (o.n.); (f) thiophenol, EtOH, reflux, 45 min.

### N-Substituted barbituric acid (step a)

Clean sodium metal (1 eq) was dissolved completely in EtOH. To this solution, diethyl malonate (1 eq) was added followed by N-substituted urea 1 (1 equivalent (eq)), after which a white precipitate formed immediately. A second portion of EtOH was then added and stirred to partially bring the precipitate into solution. The reaction mixture was then heated by microwave irradiation (µW) to 150 °C for 12 min. The resulting solid was returned to solution by addition of H₂O and then acidified with 37 % HCl and refrigerated for 3 h. The solvent was removed *in vacuo* to yield a slurry of H₂O and precipitate which was then filtered off and washed with cold H₂O to remove impurities. This process was repeated with the mother liquor (the filtrate from the washing step) to yield white crystals.

### 3-N-substituted 6-chloro-uracil 2 (step b)

To 1-N-substituted barbituric acid (1 eq) and H₂O, POCl₃ (12.5 eq) was added dropwise at 0 °C. The resulting mixture was heated by microwave irradiation at 140 °C for 5 min. Excess POCl₃ was removed via rotary evaporation and the resulting yellow oil was poured over ice water and stirred for one hour. The obtained precipitate, compound 2, was filtered and collected as a yellow powder.

### 3-N-substituted 6-hydrazino-uracil (step c)

To the 3-N-substituted 6-chlorouracil **2** (1 eq) substituted hydrazine (2.2 eq) was added in EtOH. The reaction was heated by microwave irradiation to 100 °C for 10 min. The precipitate was filtered and washed with cold EtOH to obtain pure product as white solid.

### Aldehyde hydrazones 3 (step d)

The corresponding aldehyde (1.5 eq) was added to a solution of 3-N-substituted 6-hydrazino-uracil (1 eq) in EtOH and a catalytic amount of HCl. The resulting mixture was allowed to stir for several hours. The separated precipitate was then filtered to yield the aldehyde hydrazone **3.**

### Ring closure and 4-N-oxide reduction (POG, step e and f)

Saturated aqueous solution of NaNO₂ (2 eq) was added to a solution of the aldehyde hydrazones **3** (1eq) in AcOH at 0 °C. After 10 min the reaction mixture was brought to room temperature and stirred for several hours during which time precipitate was formed. The precipitate as well as the filtrate was determined to be a mixture of both products and its 4-N-oxide. To the mixture of POG and its N-oxide in MeOH or EtOH, thiophenol (2 eq) was given and stirred at room temperature for 8-9 hours depending on substituent. After the reaction was complete, the solvent was removed via rotary evaporation and the resulting solid was recrystallized from EtOH to yield the corresponding POG derivative.

### Isolation of 4-N-oxides

For the isolation of the N-oxide proceed as described above up to step e and omit the reduction with thiophenol (step f). The mixture of POG and its corresponding 4-N-oxide was separated by normal phase chromatography in methanol/dichloromethane (MeOH/DCM) and the pure fractions were combined.

### General synthesis scheme for 4-N and 4a-substituted POG

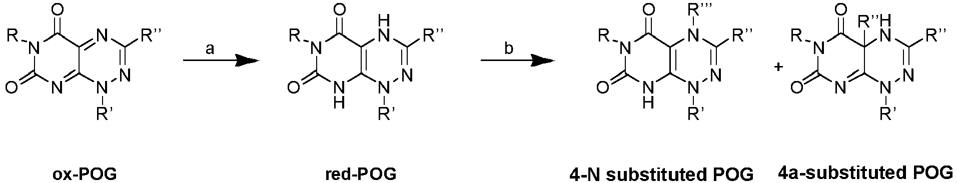

Reaction conditions: (a) thiophenol, EtOH, rt, 3 h, inert atmosphere; (b) DBU, DCM, R‴-I, 60 °C, 16 h.

### Dihydro POG (step a)

Thiophenol (2 eq) was added to a suspension of ox-POG (1 eq) in degassed EtOH under nitrogen atmosphere. The mixture was stirred for 3 h at room temperature. The dark brown precipitate was filtered off, washed three times with degassed EtOH under nitrogen and dried in vacuo to afford red-POG.

### 4-N and 4a-substituted POG (step b)

DBU (2 eq) was added to a suspension of red-POG (1 eq) in degassed CHCl₃. After 10 min alkyl iodide (1.5 eq) was added and stirred for 16 h at 60 °C. The reaction mixture was concentrated, and the remaining chloroform was washed three times each with 1M hydrochloric acid, brine, and water. The organic phase was dried over sodium sulfate and was evaporated to dryness. The residue was purified by preparative HPLC (water/MeCN) to afford 4-N substituted POG and 4a-substituted POG as yellow powders.

### General procedures for 1-N substituted POG

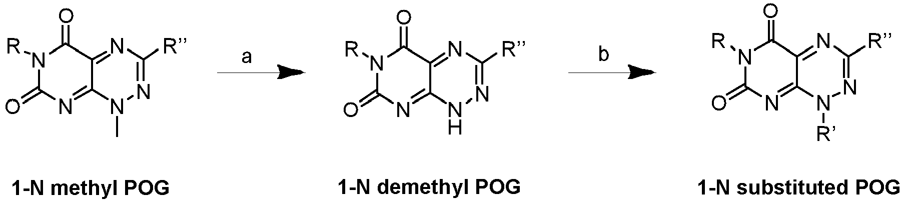

Reaction conditions: (a) DMF, 140 °C, 3 h; (b) K₂CO₃ or DBU, 1,4 dioxane, R-Br, 120 °C, 3-8 h.

### 1-N-demethylPOG (step a)

A solution of POG (18.6 mmol) in DMF was heated at 140 °C for 3 hours. Concentration of the solution *in vacuo* and recrystallisation of the residue from EtOH gave 1-N dimethyl POG.

### 1-N substituted POG (step b)

To 1-N dimethyl POG (1 eq) in 1,4-dioxane and anhydrous potassium carbonate or DBU (2 eq), an appropriate alkyl bromide (3 eq) was added and the stirring mixture was heated under reflux at 120 °C for 3-8 hours, depending on substituent. After cooling, the precipitated potassium carbonate was filtered off and the filtrate was concentrated *in vacuo.* A solution of the residue in water was extracted with ethyl acetate and the combined extracts were dried over anhydrous MgSO₄. Then the extract was evaporated *in vacuo* to leave a solid, which was recrystallized to afford the corresponding pure 1-N substituted POG derivatives.

### Synthesis protocol 3

### General synthesis scheme

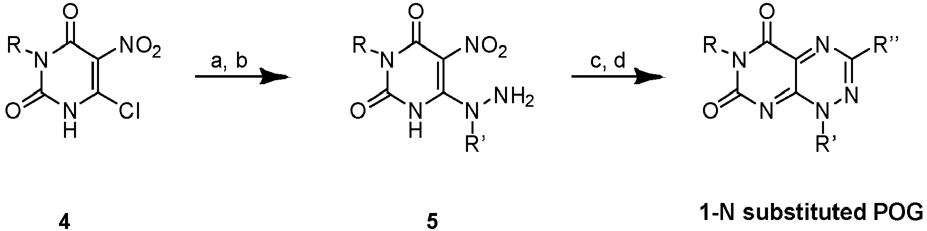

Reaction conditions: (a) DCM, R'NHNHBoc, rt, o.n.; (b) DCM, TFA, rt, o.n.; (c) R"CHO, EtOH, HCl, rt, 1 h; (d) EtOH/H₂O, Zn, NH₄Cl, 100 °C, 1 h.

### 3-N substituted 5-nitro 6-(2-N-Boc-hydrazino)-uracil (step a)

To compound 4 (1 eq) in DCM the corresponding boc-protected hydrazine (1.5 eq) was added and stirred at room temperature overnight. The formed precipitate was filtered off, washed several times with cold DCM, and dried under reduced pressure.

### 3-N substituted 5-nitro 6-hydrazino-uracil (step b)

3-N substituted 5-nitro 6-Boc-hydrazino-uracil (1 eq) was suspended in DCM and TFA (20% (v/v)) was added. The reaction mixture was stirred at room temperature until the reaction was completed. The solvent was removed under reduced pressure to obtain compound **5.**

### Aldehyde nitro hydrazones (step c)

The corresponding aldehyde (1.5 eq) was added to a solution of 3-N substituted 5-nitro 6-hydrazino-uracil **5** (1 eq) in EtOH and a catalytic amount of HCl. The resulting mixture was stirred for several hours. The separated precipitate was then filtered off to yield the corresponding aldehyde nitro hydrazone.

### 1-N substituted POG (step d)

The aldehyde nitro hydrazone (1 eq) was suspended in EtOH/H₂O and both Zn dust (4 eq) and NH₄Cl (2 eq) were added. The reaction mixture was heated for 1 h at 100 °C and then cooled to room temperature. 1N HCl-solution was added and the reaction mixture was stirred for additional 1 h at room temperature. The resulting precipitate was filtered, washed with cold EtOH and dried under reduced pressure to obtain the corresponding pure 1-N substituted POG derivatives.

### Europium-tetracycline assay for hydrogen peroxide determination

*MOPS buffer (pH 6,9)*: 3-morpholinpropylsulfonic acid (MOPS) (1.09 g, 5.20 mmol) was dissolved in 180 mL water (Milli-Q-pore). pH was adjusted to 6.9 with 10 M sodium hydroxide solution and filled up with water to 200 mL in order to obtain a 26 mM MOPS buffer.

*Eu₂Tc₂ solution:* EuCl₃ hexahydrate (9.1 mg, 24.8 µmol) and tetracycline hydrochloride (3.8 mg, 7.9 µmol) were dissolved in MOPS buffer (95 mL) each. By mixing both solutions 1:1 a solution with 131 µM EuCl₃ and 41.6 µM tetracycline in MOPS buffer was obtained.

*Hydrogen peroxide calibration solution:* 30% (w/v) hydrogen peroxide was diluted in water to 160 mM, 80 mM, 40 mM, 20 mM, 10 mM, 5 mM and 2 mM hydrogen peroxide.

*Protocol:* The assay was performed in cuvettes with a base area of 1 cm x 1 cm. Measurement of fluorescence intensity was carried out with a fluorescence spectrometer FP-6500 by Jasco at λₑₘ = 616 nm (λₑₓ = 405 nm). During measurements the solution inside the cuvette was stirred.

H₂O₂ calibration: DTT (10 µL, 200 mM; 1mM final concentration) were added to Eu₂Tc₂ (2 mL) solution in a cuvette and equilibrated by stirring for 5 min at 37 °C until the fluorescence signal was constant. The measurement was started and after 50 s H₂O₂ calibration solution (10 µL) was added. The calibration was made using 1000 µM, 800 µM, 400 µM, 200 µM, 100 µM, 50 µM 25 µM and 10 µM hydrogen peroxide. All measurements were repeated three times and a mean was calculated.

Measurements of H₂O₂ generation: protocol was the same as for calibration, but POG-4 solution (10 µL) was used instead of hydrogen peroxide. The final concentrations were 500 nM, 300 nM, 150 nM and 71 nM of POG-4. All measurements were repeated three times and a mean was calculated.

### List of references cited in the preceding sections or deemed otherwise to be relevant

1. Baell, J. B.; Holloway, G. A. New Substructure Filters for Removal of Pan Assay Interference Compounds (PAINS) From Screening Libraries and for Their Exclusion in Bioassays. J. Med. Chem. 2010, 53, 2719-2740.
2. Baell, J. B.; Nissink, J. W. M. Seven Year Itch: Pan-Assay Interference Compounds (PAINS) in 2017-Utility and Limitations. ACS Chem. Biol. 2018, 13, 36-44.
3. Baell, J.; Walters, M. A. Chemistry: Chemical Con Artists Foil Drug Discovery. Nature 2014, 513, 481-483.
4. Dahlin, J. L.; Walters, M. A. How to Triage PAINS-Full Research. Assay Drug Dev Technol 2016, 14, 168-174.
5. DE 103 01 788 A1
6. Dehaen, G.; Absillis, G.; Driesen, K.; Binnemans, K.; Parac-Vogt, T. N. (Tetracycline)Europium(III) Complex as Luminescent Probe for Hydrogen Peroxide Detection. Helv. Chim. Acta 2009, 92, 2387-2397.
7. Dürkop, A.; Wolfbeis, O. S. Nonenzymatic Direct Assay of Hydrogen Peroxide at Neutral pH Using the Eu3Tc Fluorescent Probe. J. Fluoresc. 2005, 15, 755-761.
8. Franci, G.; Sarno, F.; Nebbioso, A.; Altucci, L. Identification and Characterization of PKF118-310 as a KDM4A Inhibitor. Epigenetics 2017, 12, 198-205.
9. Hayward, D. G.; Newbatt, Y; Pickard, L.; Byrne, E.; Mao, G.; Burns, S.; Sahota, N. K.; Workman, P.; Collins, I.; Aherne, W; Fry, A. M. Identification by High-Throughput Screening of Viridin Analogs as Biochemical and Cell-Based Inhibitors of the Cell Cycle-Regulated Nek2 Kinase. J. Biomol. Screen. 2010, 15, 918-927.
10. JP H09255681 A
11. Karthikeyan, G.; Mohanraj, K.; Elango, K. P.; Girishkumar, K. Synthesis, Spectroscopic Characterization and Antibacterial Activity of Lanthanide-Tetracycline Complexes. Transition Met. Chem. 2004, 29, 86-90.
12. Latuasan, H. E.; Berends, W. On the Origin of the Toxicity of Toxoflavin. Biochim. Biophys. Acta 1961, 52, 502-508.
13. Levenberg, B.; Linton, S. N. On the Biosynthesis of Toxoflavin, an Azapteridine Antibiotic Produced by Pseudomonas Cocovenenans. J. Biol. Chem. 1966, 241, 846-852.
14. Li, X.; Li, Y; Wang, R.; Wang, Q.; Lu, L. Toxoflavin Produced by Burkholderia Gladioli From Lycoris Aurea Is a New Broad-Spectrum Fungicide. Appl. Environ. Microbiol. 2019, 85, e00106-19.
15. Light, D. R.; Walsh, C. Flavin Analogs as Mechanistic Probes ofAdrenodoxin Reductase-Dependent Electron-Transfer to the Cholesterol Side-Chain Cleavage Cytochrome-P-450 of the Adrenal-Cortex. J. Biol. Chem. 1980, 255, 4264-4277.
16. Mao, Y; Tian, W; Huang, Z. Convenient Synthesis of Toxoflavin That Targets B-Catenin/Tcf4 Signaling Activities. J. Heterocycl. Chem. 2014, 51, 594-596.
17. Nagamatsu, T.; Yamasaki, H.; Hirota, T.; Yamato, M.; Kido, Y; Shibata, M.; Yoneda, F. Syntheses of 3-Substituted 1-Methyl-6-Phenylpyrimido[5,4-E]-1,2,4-Triazine-5,7(1H,6H)-Diones (6-Phenyl Analogs of Toxoflavin) and Their 4-Oxides, and Evaluation of Antimicrobial Activity of Toxoflavins and Their Analogs. Chem. Pharm. Bull. 1993, 41, 362-368.
18. Raoof, A.; Depledge, P.; Hamilton, N. M.; Hamilton, N. S.; Hitchin, J. R.; Hopkins, G. V.; Jordan, A. M.; Maguire, L. A.; McGonagle, A. E.; Mould, D. P.; Rushbrooke, M.; Small, H. F.; Smith, K. M.; Thomson, G. J.; Turlais, F.; Waddell, I. D.; Waszkowycz, B.; Watson, A. J.; Ogilvie, D. J. Toxoflavins and Deazaflavins as the First Reported Selective Small Molecule Inhibitors of Tyrosyl-DNA Phosphodiesterase II. J. Med. Chem. 2013, 56, 6352-6370.
19. Schrenkhammer, P.; Rosnizeck, I. C.; Duerkop, A.; Wolfbeis, O. S.; Schaferling, M. Time-Resolved Fluorescence-Based Assay for the Determination of Alkaline Phosphatase Activity and Application to the Screening of Its Inhibitors. J. Biomol. Screen. 2007, 13, 9-16.
20. Stewart, R.; Srinivasan, R.; Gumbley, S. J. Chemical-Reactivity of Lumazine Derivatives -Acid-Base and Redox Reactions of a Number of Lumazines, Deazalumazines, and Isoalloxazines - an Unusual Effect of Methyl Substitution. Can. J. Chem. 1981, 59, 2755-2765.
21. Todorovic, N.; Giacomelli, A.; Hassell, J. A.; Frampton, C. S.; Capretta, A. Microwave-Assisted Synthesis of 3-Aryl-Pyrimido[5,4-E][1,2,4]Triazine-5,7(1H,6H)-Dione Libraries: Derivatives of Toxoflavin. Tetrahedron Lett. 2010, 51, 6037-6040.
22. Vidler, L. R.; Watson, I. A.; Margolis, B. J.; Cummins, D. J.; Brunavs, M. Investigating the Behavior of Published PAINS Alerts Using a Pharmaceutical Company Data Set. ACS Med. Chem. Lett. 2018, 9, 792-796.
23. Walsh, C.; Fisher, J.; Spencer, R.; Graham, D. W.;Ashton, W. T.; Brown, J. E.; Brown, R. D.; Rogers, E. F. Chemical and Enzymatic Properties of Riboflavin Analogs. Biochemistry 1978, 17, 1942-1951.
24. Wei, W; Chua, M.-S.; Grepper, S.; So, S. Small Molecule Antagonists ofTcf4/Beta-Catenin Complex Inhibit the Growth of HCC Cells in Vitro and in Vivo. Int. J. Cancer 2010, 126, 2426-2436.
25. WO 2010/014798 A2
26. WO 2010/072807 A2
27. WO 2012/006104 A2
28. WO 2016/118014 A2
29. WO 2018/140762 A1
30. Wolfbeis, O. S.; Dürkop, A.; Wu, M.; Lin, Z. A Europium-lon-Based Luminescent Sensing Probe for Hydrogen Peroxide. Angew. Chem. Int. Ed. 2002, 41, 4495-4498.
31. Wu, M.; Lin, Z.; Schäferling, M.; Dürkop, A.; Wolfbeis, O. S. Fluorescence Imaging of the Activity of Glucose Oxidase Using a Hydrogen-Peroxide-Sensitive Europium Probe. Anal. Biochem. 2005, 340, 66-73.
32. Yoneda, F., Shinomura, K., & Nishigaki, S. A convinient synthesis of toxoflavins and toxoflavin-n-oxides. Tetrahedron Letters 1971, 12(13), 851-854.
33. Yoneda, F., & Nagamatsu, T. Transformation of toxoflavin into fervenulin via 1-demethyltoxoflavin. Tetrahedron Letters 1973, 14(17), 1577-1580.
34. Yoneda, F.; Nagamatsu, T. A Convenient Synthesis of Toxoflavins, Toxoflavin 4-Oxides and 1-Demethyltoxoflavins. Chem. Pharm. Bull. 1975, 23, 2001-2009.
35. Zhou, Y; Wei, L.; Brady, T. P.; Redddy, P. S. M. M.; Nguyen, T.; Chen, J.; Au, Q.; Sang Yoon, II; Yip, G.; Bin Zhang; Barber, J. R.; Ng, S. C. Pyrimido[5,4-E][1,2,4]Triazine-5,7(1H,6H)-Dione Derivatives as Novel Small Molecule Chaperone Amplifiers. Bioorg. Med. Chem. Lett. 2009, 19, 4303-4307.
36. Lee, S., Yu, Y., Trimpert, J., Benthani, F., Mairhofer, M., Richter-Pechanska, P., ... & Schmitt, C. A. Virus-induced senescence is a driver and therapeutic target in COVID-19. Nature, 2021, 599(7884), 283-289.
37. Milanovic, M., Fan, D. N., Belenki, D., Däbritz, J. H. M., Zhao, Z., Yu, Y., ... & Schmitt, C. A. Senescence-associated reprogramming promotes cancer stemness. Nature 2018, 553(7686), 96-100.
38. Dörr, J. R., Yu, Y., Milanovic, M., Beuster, G., Zasada, C., Däbritz, J. H. M., ... & Schmitt, C. A. Synthetic lethal metabolic targeting of cellular senescence in cancer therapy. Nature 2013, 501(7467), 421-425.
39. Braig, M., Lee, S., Loddenkemper, C., Rudolph, C., Peters, A. H., Schlegelberger, B., ... & Schmitt, C. A. Oncogene-induced senescence as an initial barrier in lymphoma development. Nature 2005, 436(7051), 660-665.

## Claims

1. Compound according to general formula (I), a derivative of such compound according to any of general formulae (II), (III), or (IV), or a pharmaceutically acceptable salt or hydrate of such compound or derivative, for in-vivo use as senolytic: 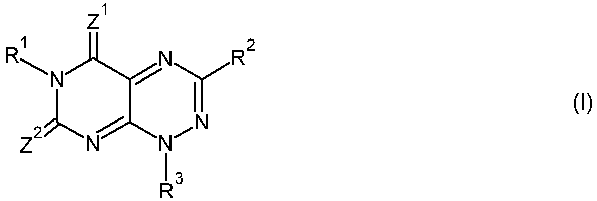 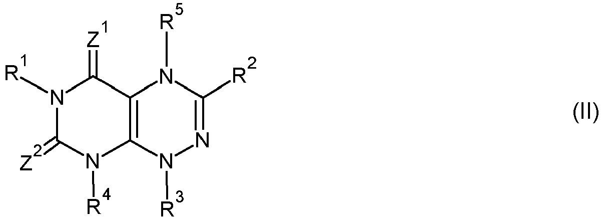 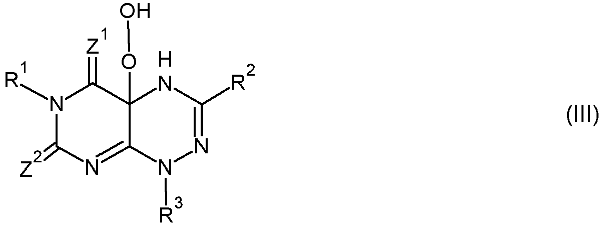 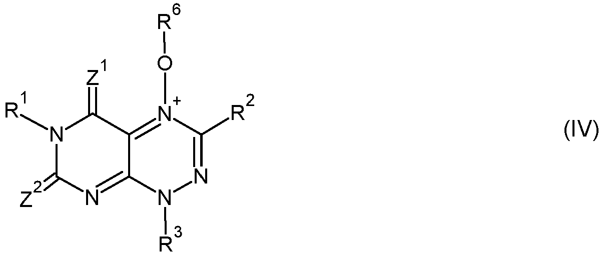 wherein
R¹ = H, C₁-C₁₀ alkyl, C₃-C₂₀ cycloalkyl, heteroatom-substituted C₃-C₂₀ cycloalkyl, CF₃, CF₂H, CFH₂, optionally substituted piperidine, C₁-C₁₀ alkyl carrying a primary, secondary or tertiary amino substituent, OH, CH₂-O-galactosyl, optionally substituted C₆-C₂₀ aryl, or optionally substituted C₅-C₂₀ heteroaryl,
R² = optionally substituted C₁-C₁₀ alkyl, optionally substituted C₇-C₃₀ alkyl aryl, optionally substituted C₆-C₃₀ alkyl heteroaryl, optionally substituted C₆-C₂₀ aryl, optionally substituted C₆-C₂₀ aryl nitro, optionally substituted C₂-C₁₀ alkenyl, optionally substituted C₈-C₃₀ alkenyl aryl, optionally substituted C₈-C₃₀ alkenyl aryl nitro, optionally substituted C₇-C₃₀ alkenyl heteroaryl, optionally substituted C₂-C₁₀ alkinyl, optionally substituted C₈-C₃₀ alkinyl aryl, optionally substituted C₇-C₃₀ alkinyl heteroaryl, optionally substituted C₁₁-C₃₀ heteroaryl aryl, optionally substituted C₉-C₃₀ cyclyl aryl, optionally substituted C₈-C₃₀ heterocyclyl aryl, optionally substituted C₃-C₂₀ cycloalkyl, halogen, OH, C₁-C₁₀ alkoxy, C₆-C₂₀ aryloxy, amino, substituted amino, CN, carboxy, optionally substituted C₁-C₁₀ alkyl carboxy, optionally substituted C₆-C₂₀ aryl-carboxy, carbamido, optionally substituted C₅-C₂₀ heteroaryl, optionally substituted C₂-C₂₀ heterocyclyl, heteroatom-substituted C₆-C₂₀ cycloalkyl, optionally substituted SH, sulfonamido, or sulfone,
R³ = C₁-C₁₀ alkyl, C₃-C₂₀ cycloalkyl, heteroatom-substituted C₃-C₂₀ cycloalkyl, CF₃, CF₂H, CFH₂, optionally substituted piperidine, C₁-C₁₀ alkyl carrying a primary, secondary or tertiary amino substituent, OH, CH₂-O-galactosyl, optionally substituted C₆-C₂₀ aryl, or optionally substituted C₅-C₂₀ heteroaryl,
R⁴, R⁵ = independently from each other H, C₁-C₁₀ alkyl, C₃-C₂₀ cycloalkyl, heteroatom-substituted C₃-C₂₀ cycloalkyl, CF₃, CF₂H, CFH₂, optionally substituted piperidine, C₁-C₁₀ alkyl carrying a primary, secondary or tertiary amino substituent, OH, CH₂-O-galactosyl, optionally substituted C₆-C₂₀ aryl, or optionally substituted C₅-C₂₀ heteroaryl, or a protecting group chosen from i) tert-butyloxycarbonyl, ii) trityl, iii) acetyl, iv) C₁-C₁₀ acyl residues, and v) monosubstituted or disubstituted compounds according to general formula (II), wherein R⁴ and R⁵ denote H,
R⁶ = absent or a protecting group chosen from C₁-C₁₀ acyl, acetyl, ester residues, benzoyl, benzyl, trityl, and ether residues, and
Z¹, Z² = independently from each other O or S.

2. Compound for use according to claim 1, **characterized in that** residue R² is a residue according to any of the following formulae, wherein the dashed line indicates a bond by which the residue R² is covalently bound to the triazine ring of the compound according to general formulae (I), (II), (III), or (IV): 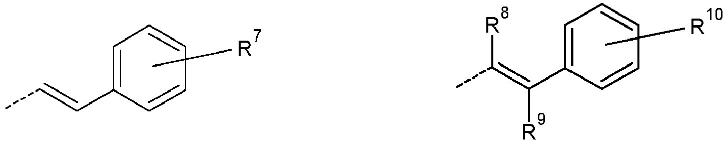 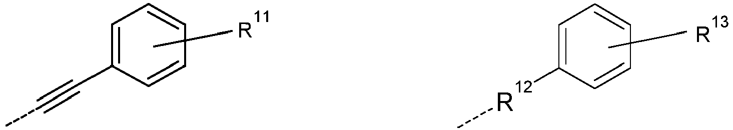 wherein
R⁷, R¹⁰, R¹¹, R¹³ = independently from each other H, NO₂, optionally substituted C₁-C₁₀ alkyl, optionally substituted C₂-C₁₀ alkenyl, optionally substituted C₃-C₂₀ cycloalkyl, heteroatom-substituted C₆-C₂₀ cycloalkyl, halogen, OH, C₁-C₁₀ alkoxy, C₆-C₂₀ aryloxy, amino, substituted amino, CN, carboxy, optionally substituted C₁-C₁₀ alkyl carboxy, optionally substituted C₆-C₂₀ aryl-carboxy, carbamido, optionally substituted C₅-C₂₀ heteroaryl, optionally substituted C₂-C₂₀ heterocyclyl, optionally substituted C₆-C₂₀ aryl, optionally substituted SH, sulfonamido, or sulfone,
R⁸, R⁹ = independently from each other H, optionally substituted C₁-C₁₀ alkyl, optionally substituted C₂-C₁₀ alkenyl, optionally substituted C₃-C₂₀ cycloalkyl, heteroatom-substituted C₆-C₂₀ cycloalkyl, halogen, OH, C₁-C₁₀ alkoxy, C₆-C₂₀ aryloxy, amino, substituted amino, CN, carboxy, optionally substituted C₁-C₁₀ alkyl carboxy, optionally substituted C₆-C₂₀ aryl-carboxy, carbamido, optionally substituted C₅-C₂₀ heteroaryl, optionally substituted C₂-C₂₀ heterocyclyl, optionally substituted C₆-C₂₀ aryl, optionally substituted SH, sulfonamido, or sulfone, CF₃, CF₂H, or CFH₂, and
R¹² = independently from each other optionally substituted C₁-C₁₀ alkyl, optionally substituted C₂-C₁₀ alkenyl, optionally substituted C₂-C₁₀ alkinyl, optionally substituted C₆-C₂₀ aryl, optionally substituted C₅-C₂₀ heteroaryl, optionally substituted C₃-C₂₀ cycloalkyl, optionally substituted C₃-C₂₀ cyclyl, or optionally substituted C₂-C₂₀ heterocyclyl.

3. Compound for use according to claim 1 or 2, **characterized in that** residue R² is a residue according to any of the following formulae, wherein the dashed line indicates a bond by which the residue R² is covalently bound to the triazine ring of the compound according to general formulae (I), (II), (III), or (IV): 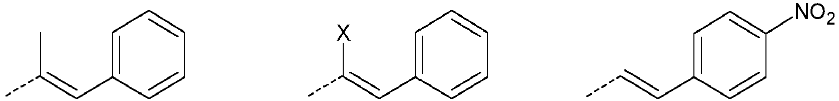 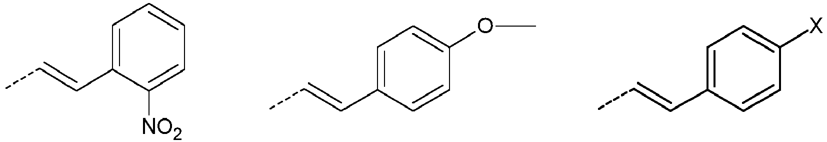 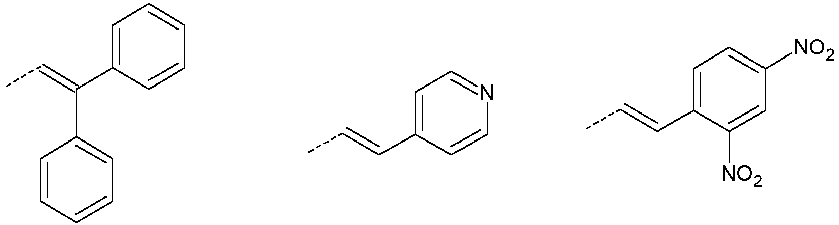 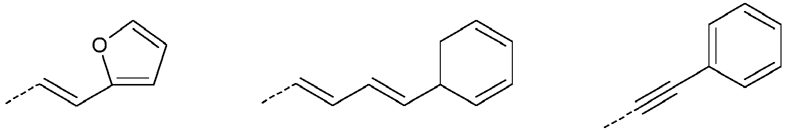 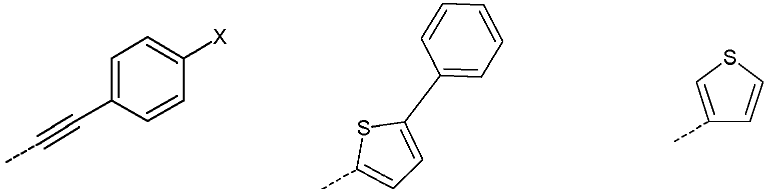 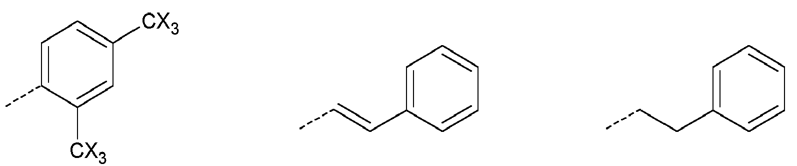 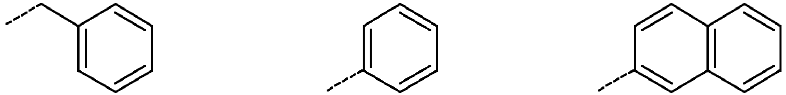 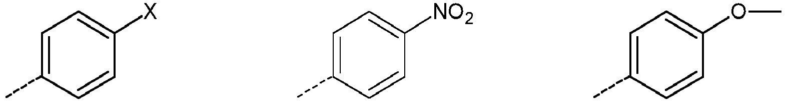 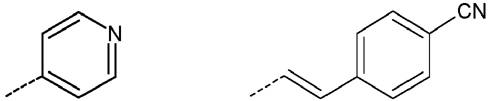 wherein
X = F, Cl, Br, or I.

4. Compound for use according to any of the preceding claims, **characterized in that** residue R² is a residue according to any of the following formulae, wherein the dashed line indicates a bond by which the residue R² is covalently bound to the triazine ring of the compound according to general formulae (I), (II), (III), or (IV): 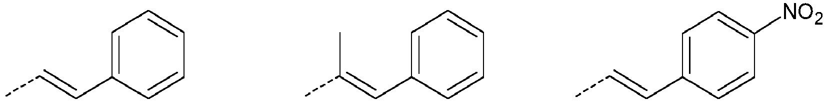 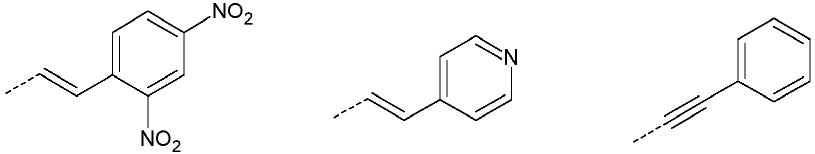 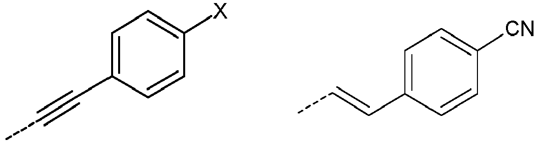 wherein
X = F, Cl, Br, or I.

5. Compound for use according to any of the preceding claims, **characterized in that** residue R¹ is a methyl residue or a hydrogen.

6. Compound for use according to any of the preceding claims, **characterized in that** residue R³ is a methyl residue.

7. Compound for use according to any of the preceding claims, **characterized in that** residue R¹ is a methyl residue or a hydrogen and residue R³ is chosen from methyl, ethyl, isopropyl, and phenyl.

8. Compound for use according to any of the preceding claims, **characterized in that** residue R¹ is a methyl residue or a hydrogen and R³ is a methyl residue.

9. Compound for use according to any of the preceding claims, **characterized in that** the compound is for use in treating cancer that has previously been treated with a chemotherapeutic agent.

10. In-vitro method for senolysis, the method comprising subjecting senescent cells to a compound according to general formula (I), to a derivative of such compound according to any of general formulae (II), (III), or (IV), or to a pharmaceutically acceptable salt or hydrate of such compound or derivative: 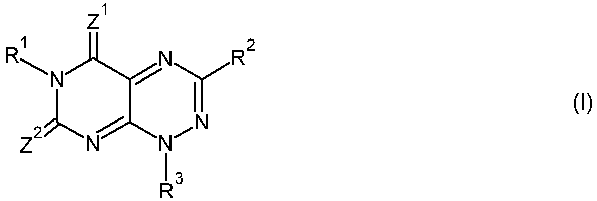 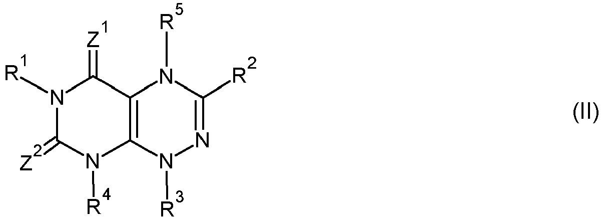 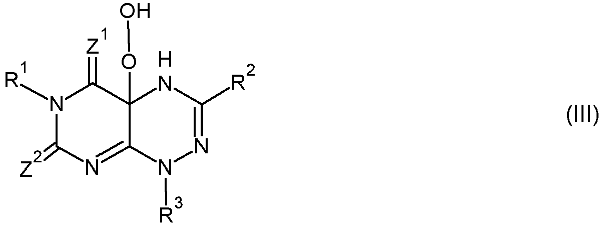 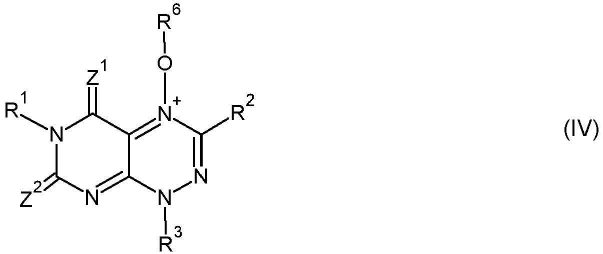 wherein
R¹ = H, C₁-C₁₀ alkyl, C₃-C₂₀ cycloalkyl, heteroatom-substituted C₃-C₂₀ cycloalkyl, CF₃, CF₂H, CFH₂, optionally substituted piperidine, C₁-C₁₀ alkyl carrying a primary, secondary or tertiary amino substituent, OH, CH₂-O-galactosyl, optionally substituted C₆-C₂₀ aryl, or optionally substituted C₅-C₂₀ heteroaryl,
R² = optionally substituted C₁-C₁₀ alkyl, optionally substituted C₇-C₃₀ alkyl aryl, optionally substituted C₆-C₃₀ alkyl heteroaryl, optionally substituted C₆-C₂₀ aryl, optionally substituted C₆-C₂₀ aryl nitro, optionally substituted C₂-C₁₀ alkenyl, optionally substituted C₈-C₃₀ alkenyl aryl, optionally substituted C₈-C₃₀ alkenyl aryl nitro, optionally substituted C₇-C₃₀ alkenyl heteroaryl, optionally substituted C₂-C₁₀ alkinyl, optionally substituted C₈-C₃₀ alkinyl aryl, optionally substituted C₇-C₃₀ alkinyl heteroaryl, optionally substituted C₁₁-C₃₀ heteroaryl aryl, optionally substituted C₉-C₃₀ cyclyl aryl, optionally substituted C₈-C₃₀ heterocyclyl aryl, optionally substituted C₃-C₂₀ cycloalkyl, halogen, OH, C₁-C₁₀ alkoxy, C₆-C₂₀ aryloxy, amino, substituted amino, CN, carboxy, optionally substituted C₁-C₁₀ alkyl carboxy, optionally substituted C₆-C₂₀ aryl-carboxy, carbamido, optionally substituted C₅-C₂₀ heteroaryl, optionally substituted C₂-C₂₀ heterocyclyl, heteroatom-substituted C₆-C₂₀ cycloalkyl, optionally substituted SH, sulfonamido, or sulfone,
R³ = C₁-C₁₀ alkyl, C₃-C₂₀ cycloalkyl, heteroatom-substituted C₃-C₂₀ cycloalkyl, CF₃, CF₂H, CFH₂, optionally substituted piperidine, C₁-C₁₀ alkyl carrying a primary, secondary or tertiary amino substituent, OH, CH₂-O-galactosyl, optionally substituted C₆-C₂₀ aryl, or optionally substituted C₅-C₂₀ heteroaryl,
R⁴, R⁵ = independently from each other H, C₁-C₁₀ alkyl, C₃-C₂₀ cycloalkyl, heteroatom-substituted C₃-C₂₀ cycloalkyl, CF₃, CF₂H, CFH₂, optionally substituted piperidine, C₁-C₁₀ alkyl carrying a primary, secondary or tertiary amino substituent, OH, CH₂-O-galactosyl, optionally substituted C₆-C₂₀ aryl, or optionally substituted C₅-C₂₀ heteroaryl, or a protecting group chosen from i) tert-butyloxycarbonyl, ii) trityl, iii) acetyl, iv) C₁-C₁₀ acyl residues, and v) monosubstituted or disubstituted compounds according to general formula (II), wherein R⁴ and R⁵ denote H,
R⁶ = absent or a protecting group chosen from C₁-C₁₀ acyl, acetyl, ester residues, benzoyl, benzyl, trityl, and ether residues and
Z¹, Z² = independently from each other O or S.

11. Compound according to general formula (I), a derivative of such compound according to any of general formulae (II), (III), or (IV), or a pharmaceutically acceptable salt or hydrate of such compound or derivative: 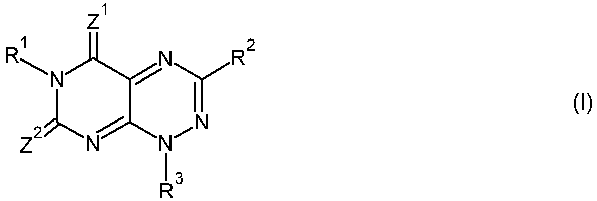 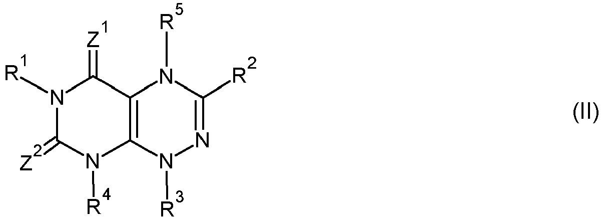 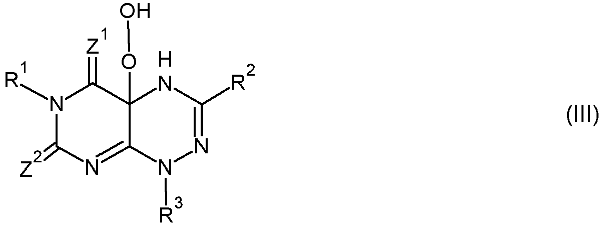 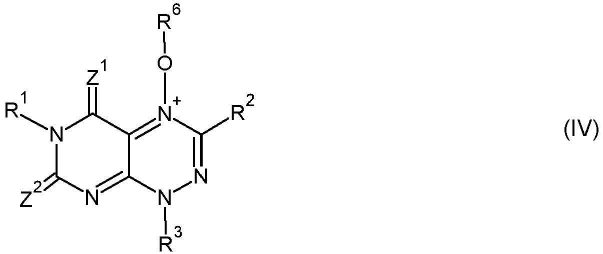 wherein
R¹ = H, C₁-C₁₀ alkyl, C₃-C₂₀ cycloalkyl, heteroatom-substituted C₃-C₂₀ cycloalkyl, CF₃, CF₂H, CFH₂, optionally substituted piperidine, C₁-C₁₀ alkyl carrying a primary, secondary or tertiary amino substituent, OH, CH₂-O-galactosyl, optionally substituted C₆-C₂₀ aryl, or optionally substituted C₅-C₂₀ heteroaryl,
R² = optionally substituted C₁-C₁₀ alkyl, optionally substituted C₇-C₃₀ alkyl aryl, optionally substituted C₆-C₃₀ alkyl heteroaryl, optionally substituted C6-C20 aryl, optionally substituted C6-C20 aryl nitro, optionally substituted C₂-C₁₀ alkenyl, optionally substituted C₈-C₃₀ alkenyl aryl, optionally substituted C₈-C₃₀ alkenyl aryl nitro, optionally substituted C₇-C₃₀ alkenyl heteroaryl, optionally substituted C₂-C₁₀ alkinyl, optionally substituted C₈-C₃₀ alkinyl aryl, optionally substituted C₇-C₃₀ alkinyl heteroaryl, optionally substituted C₁₁-C₃₀ heteroaryl aryl, optionally substituted C₉-C₃₀ cyclyl aryl, optionally substituted C₈-C₃₀ heterocyclyl aryl, optionally substituted C₃-C₂₀ cycloalkyl, halogen, OH, C₁-C₁₀ alkoxy, C₆-C₂₀ aryloxy, amino, substituted amino, CN, carboxy, optionally substituted C₁-C₁₀ alkyl carboxy, optionally substituted C₆-C₂₀ aryl-carboxy, carbamido, optionally substituted C₅-C₂₀ heteroaryl, optionally substituted C₂-C₂₀ heterocyclyl, heteroatom-substituted C₆-C₂₀ cycloalkyl, optionally substituted SH, sulfonamido, or sulfone,
R³ = C₁-C₁₀ alkyl, C₃-C₂₀ cycloalkyl, heteroatom-substituted C₃-C₂₀ cycloalkyl, CF₃, CF₂H, CFH₂, optionally substituted piperidine, C₁-C₁₀ alkyl carrying a primary, secondary or tertiary amino substituent, OH, CH₂-O-galactosyl, optionally substituted C₆-C₂₀ aryl, or optionally substituted C₅-C₂₀ heteroaryl,
R⁴, R⁵ = independently from each other H, C₁-C₁₀ alkyl, C₃-C₂₀ cycloalkyl, heteroatom-substituted C₃-C₂₀ cycloalkyl, CF₃, CF₂H, CFH₂, optionally substituted piperidine, C₁-C₁₀ alkyl carrying a primary, secondary or tertiary amino substituent, OH, CH₂-O-galactosyl, optionally substituted C₆-C₂₀ aryl, or optionally substituted C₅-C₂₀ heteroaryl, or a protecting group chosen from i) tert-butyloxycarbonyl, ii) trityl, iii) acetyl, iv) C₁-C₁₀ acyl residues, and v) monosubstituted or disubstituted compounds according to general formula (II), wherein R⁴ and R⁵ denote H,
R⁶ = absent or a protecting group chosen from C₁-C₁₀ acyl, acetyl, ester residues, benzoyl, benzyl, trityl, and ether residues, and
Z¹, Z² = independently from each other O or S,
with the proviso that residue R² does not comply with any of the following formulae if the indicated further condition is fulfilled, wherein the dashed line indicates a bond by which the residue R² is covalently bound to the triazine ring of the compound according to general formulae (I), (II), (III), or (IV):
i) 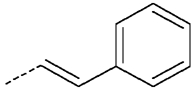 if residue R¹ is a methyl residue and residue R³ is a methyl or ethyl residue,
ii) 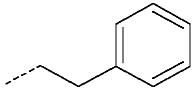 if residue R¹ is a methyl residue and residue R³ is a methyl residue,
iii) 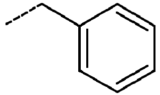 if residue R¹ is a methyl residue and residue R³ is a methyl residue,
iv) 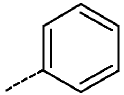 if residue R¹ is a methyl residue and residue R³ is a methyl residue,
v) 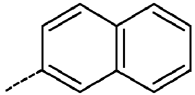 if residue R¹ is a methyl residue and residue R³ is a methyl residue,
vi) 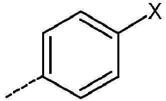 if residue R¹ is a methyl residue and residue R³ is a methyl residue wherein X denotes F, Cl, Br, or I, and
vii) 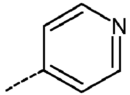 if residue R¹ is a methyl residue and residue R³ is a methyl residue.

12. Compound according to claim 11, **characterized in that** residue R² is a residue according to any of the following formulae, wherein the dashed line indicates a bond by which the residue R² is covalently bound to the triazine ring of the compound according to general formulae (I), (II), (III), or (IV): 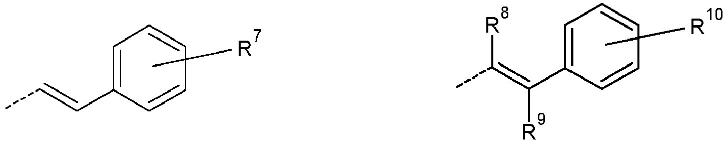 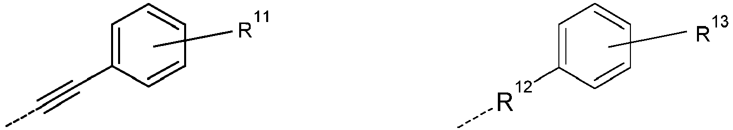 wherein
R⁷, R¹⁰, R¹¹, R¹³ = independently from each other H, NO₂, optionally substituted C₁-C₁₀ alkyl, optionally substituted C₂-C₁₀ alkenyl, optionally substituted C₃-C₂₀ cycloalkyl, halogen, OH, C₁-C₁₀ alkoxy, C₆-C₂₀ aryloxy, amino, substituted amino, CN, carboxy, optionally substituted C₁-C₁₀ alkyl carboxy, optionally substituted C₆-C₂₀ aryl-carboxy, carbamido, optionally substituted C₅-C₂₀ heteroaryl, optionally substituted C₂-C₂₀ heterocyclyl, heteroatom-substituted C₆-C₂₀ cycloalkyl, optionally substituted C₆-C₂₀ aryl, optionally substituted SH, sulfonamido, or sulfone,
R⁸, R⁹ = independently from each other H, optionally substituted C₁-C₁₀ alkyl, optionally substituted C₂-C₁₀ alkenyl, optionally substituted C₃-C₂₀ cycloalkyl, halogen, OH, C₁-C₁₀ alkoxy, C₆-C₂₀ aryloxy, amino, substituted amino, CN, carboxy, optionally substituted C₁-C₁₀ alkyl carboxy, optionally substituted C₆-C₂₀ aryl-carboxy, carbamido, optionally substituted C₅-C₂₀ heteroaryl, optionally substituted C₂-C₂₀ heterocyclyl, heteroatom-substituted C₆-C₂₀ cycloalkyl, optionally substituted C₆-C₂₀ aryl, optionally substituted SH, sulfonamido, or sulfone, CF₃, CF₂H, or CFH₂, and
R¹² = independently from each other optionally substituted C₁-C₁₀ alkyl, optionally substituted C₂-C₁₀ alkenyl, optionally substituted C₂-C₁₀ alkinyl, optionally substituted C₆-C₂₀ aryl, optionally substituted C₅-C₂₀ heteroaryl, optionally substituted C₃-C₂₀ cycloalkyl, optionally substituted C₃-C₂₀ cyclyl, or optionally substituted C₂-C₂₀ heterocyclyl.

13. Compound according to claim 11 or 12, **characterized in that** that residue R² does not comply with any of the following formulae, wherein the dashed line indicates a bond by which the residue R² is covalently bound to the triazine ring of the compound according to general formulae (I), (II), (III), or (IV): 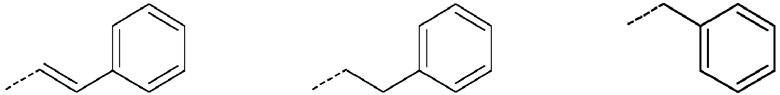 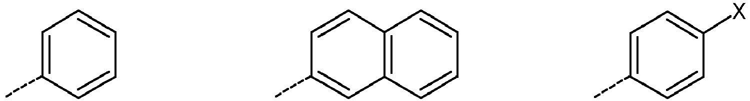 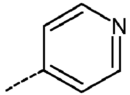 wherein
X = F, Cl, Br, or I.

14. Compound according to claim 11 or 13, **characterized in that** that residue R² is a residue according to any of the following formulae, wherein the dashed line indicates a bond by which the residue R² is covalently bound to the triazine ring of the compound according to general formulae (I), (II), (III), or (IV): 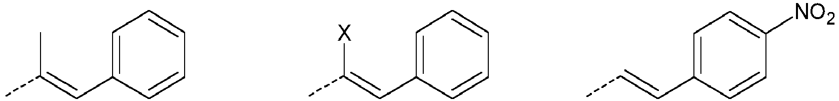 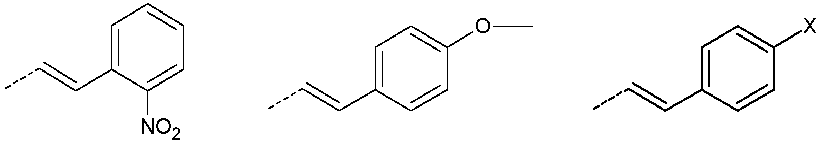 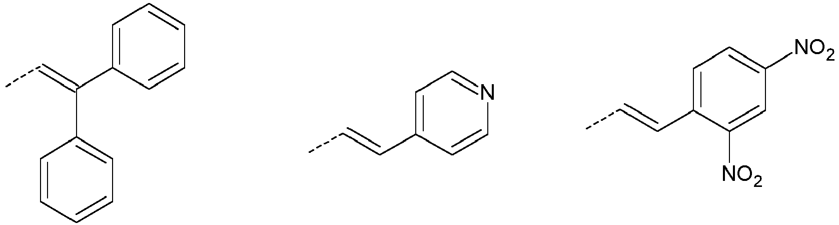 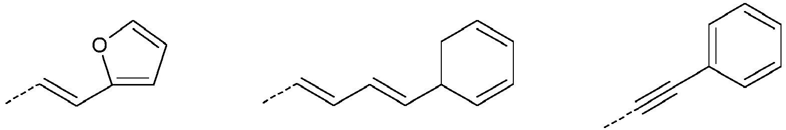 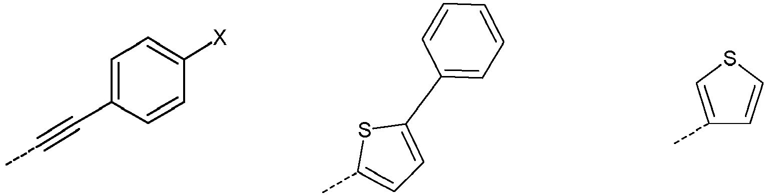 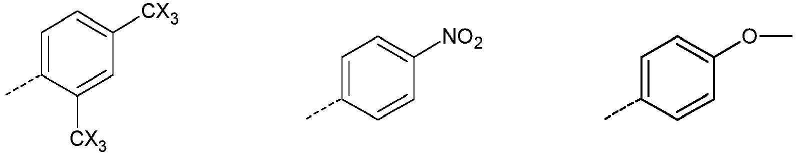 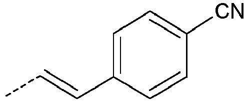 wherein
X = F, Cl, Br, or I.

15. Medicament, comprising at least one compound according to any of claims 11 to 14.
